# EUROPÄISCHE PATENTANMELDUNG

(11) **EP 1 418 230 A2**
(43) Veröffentlichungstag der Anmeldung: **12.05.2004**
(21) Anmeldenummer: 03025190.4
(22) Anmeldetag: 04.11.2003
(51) Int. Cl.: C12N 9/84, C12N 15/63, C12N 1/21

(54) **Gentechnisch veränderte Penicillinamidase und Verfahren zu ihrer Herstellung**

(30) Priorität: 05.11.2002 DE 10251747
(71) Anmelder: TUHH-Technologie GmbH, 21079 Hamburg (DE); Technische Universität Hamburg-Harburg, 21073 Hamburg (DE)
(72) Erfinder: Kasche, Volker, Prof. Dr., 28359 Bremen (DE); Ignatova, Zoya, 22769 Hamburg (DE); Galunsky, Boris, 21073 Hamburg (DE)
(74) Vertreter: Pohl, Manfred, Dr.

(57) **Zusammenfassung**

Die Erfindung betrifft eine gentechnisch veränderte Penicillinamidase und Verfahren zu ihrer Herstellung. Bei der erfindungsgemäßen Penicillinamidase ist die vollständige Abspaltung eines Linker-Peptids verzögert oder blockiert, so daß das Linker-Peptid oder ein Teil davon kovalent an der Penicillinamidase gebunden bleibt, was zu einer deutlichen Erhöhung der Enzymaktivität der Penicillinamidase gegenüber dem Wildtyp-Enzym ohne das Linker-Peptid oder dessen Teil führt.

## Beschreibung

Die Erfindung betrifft eine gentechnisch veränderte Penicillinamidase mit gegenüber dem Wildtyp erhöhter Aktivität, eine Pro-Penicillinamidase, aus der die Penicillinamidase in vivo hergestellt wird sowie Nukleinsäuren, die für die Penicillinamidase oder Pro-Penicillinamidase kodieren. Darüber hinaus betrifft die Erfindung auch ein Verfahren zur Herstellung eines Enzyms mit gegenüber dem Wildtyp veränderter Aktivität, ein Verfahren zur Herstellung von 6-Aminopenicillansäure (6-APA) bzw. 7-Aminodesacetoxycephalosporansäure (7-ADCA), eine Zusammensetzung und deren Verwendung zur Erhöhung der Aktivität einer Penicillinamidase sowie Vektoren und Wirtszellen, die Nukleinsäuren enthalten, die für die Penicillinamidase oder Pro-Penicillinamidase kodieren.

Die Penicillinamidase (PA, EC 3.5.1.11), auch Penicillinacylase (PAC) genannt, wird in industriellem Maßstab verwendet, um Penicillin G oder 3-Desacetoxycephalosporin zu Phenylessigsäure und 6-Aminopenicillansäure (6-APA) bzw. 7-Aminodesacetoxycephalosporansäure (7-ADCA) zu hydrolysieren. 6-APA und 7-ADCA werden für die Produktion von semisynthetischen Penicillinen und Cephalosporinen (β-Laktam-Antibiotika) eingesetzt. Das Enzym kann auch zur gleichgewichts- oder kinetisch kontrollierten Synthese dieser semisynthetischen ß-Laktam-Antibiotika genutzt werden (Kasche, V., Enz. Microb. Technol. 8, 4, 1986).

Verschiedene Organismen sind geeignete Quellen für die Penicillinamidase (Huang, H.T. et al., Appl. Microb., 11, 1-23, 1963). Im industriellen Maßstab sind bisher die Enzyme aus u.a. *Escherichia coli* , *Kluyvera citrophila, Providencia rettgeri, Bacillus megaterium* und *Alcaligenes faecalis* eingesetzt worden. Penicillinamidaseaktivität wurde in *A. faecalis* bereits 1960 entdeckt (Claveridge, C.A. et al., Nature 4733, 237-238, 1969). Das Gen für die Penicillinamidase aus diesem Organismus ist allerdings erst Anfang der 1990er Jahre durch die US 5,168,048 bekannt geworden. Die Penicillinamidase aus *A. faecalis* weist im Vergleich zu der Penicillinamidase aus *E. coli* eine bessere Temperatur- und pH-Stabilität auf, insbesondere bei pH-Werten über 8.0 (Verhaert, R.M. et al., Appl. Environ. Microbiol. 63, 3412-3418, 1997). Damit können höhere Hydrolyseausbeuten erreicht werden, da diese mit pH-Werten im Bereich von 7-9 steigen.

Für alle Penicillinamidasen aus den oben genannten Organismen gilt, daß das entsprechende Gen für eine Polypeptidkette (Prä-Pro-Penicillinamidase) mit einem Signalpeptid kodiert. Das Signalpeptid wird bei dem Transport durch die Zellmembran mittels Hydrolyse entfernt (Virden, R. Biotechnol. Gen Eng. Rev. 8, 189-218, 1990). Die so erhaltene Pro-Penicillinamidase weist ein Linker-Peptid zwischen der zukünftigen A-Kette (alpha-Untereinheit) und der zukünftigen B-Kette (beta-Untereinheit) auf. Durch eine intramolekulare autoproteolytische Spaltung in der Pro-Penicillinamidase entsteht das katalytisch aktive N-terminale Serin der B-Kette (Molekulargewicht ≈ 62 kDa, Kasche, V. et al. Biochim. Biophys. Acta 1433, 76-86, 1999). Bei *A. faecalis* erfolgt die Spaltung zwischen Ala₂₃₉-Ser₂₄₀ (Numerierung bezogen auf die Pro-PA, SEQ ID NO: 1, SEQ ID NO: 2). Die darauf folgenden Hydrolysen zwischen dem C-Terminus des Linkers und dem zukünftigen C-Terminus der A-Kette des Enzyms (≈ 22 kDa) führen zur vollständigen Entfernung des Linkers (Hewitt, L. et al., J. Mol.

Biol. 302, 887-898, 2000; Kasche, V. et al., Biochim. Biophys. Acta 1433, 76-86, 1999). Die Länge des Linkers variiert von 54 bei der Penicillinamidase aus *E. coli* bis zu 37 Aminosäuren bei dem Enzym aus *A. faecalis* (Verhaert, R.M. et al., Appl. Environ. Microbiol. 63, 3412-3418, 1997).

Die Penicillinamidase ist von großer Bedeutung für die Herstellung von Beta-Laktam-Antibiotika. Dabei ist es wünschenswert, Enzyme mit möglichst großer Stabilität und Aktivität einzusetzen, um den Herstellungsprozeß im technischen Maßstab zu optimieren. Diesen Anforderungen werden die bisher verwendeten Wildtyp-Enzyme häufig nicht ausreichend gerecht. Aufgabe der Erfindung ist es daher, eine Penicillinamidase bereitzustellen, die stabil ist und eine gegenüber dem Wildtyp-Enzym erhöhte Aktivität aufweist. Weiter ist es Aufgabe der Erfindung, das Enzym in einer Form verfügbar zu machen, die ein einfaches Handling erlaubt und es auch im technischen Maßstab einsetzbar macht. Darüber hinaus ist es Aufgabe der Erfindungn, ein Verfahren bereitzustellen, mit dessen Hilfe ein Enzym mit gegenüber dem Wildtyp-Enzym veränderter Aktivität hergestellt werden kann.

Die Lösung der Aufgabe wird durch den Gegenstand des Anspruchs 1 und die Gegenstände der nebengeordneten Ansprüche gelöst.

Es wurde überraschend gefunden, daß die Enzymaktivität der Penicillinamidase in Gegenwart des Linker-Peptids bzw. von Teilen des Linker-Peptids gegenüber der Aktivität des gereinigten Enzyms erhöht ist. Es wurde darüber hinaus gefunden, daß ein Enzym, das das Linker-Peptid oder einen Teil davon kovalent an eine Peptid-Einheit des Enzyms gebunden enthält, ebenfalls eine erhöhte Aktivität gegenüber einem Enzym aufweist, das die gleiche Peptid-Einheit, aber ohne das Linker-Peptid oder einen Teil davon, enthält.

Die erfindungsgemäße Penicillinamidase weist daher das Linker-Peptid oder einen Teil davon kovalent an eine Peptid-Einheit der Penicillinamidase gebunden auf.

Bevorzugt stammt das Linker-Peptid oder ein Teil des Linker-Peptids von einem Vorläufer-Enzym (Pro-Enzym), bei dem das Linker-Peptid in vivo die Aminosäurekette der Peptid-Einheit mit der Aminosäurekette einer weiteren Peptid-Einheit verbindet.

Besonders bevorzugt ist bei der erfindungsgemäßen Penicillinamidase die Aminosäuresequenz des Linker-Peptids derart gegenüber dem Linker-Peptid des Wildtyps modifiziert, daß die ansonsten stattfindende Abspaltung des Linker-Peptids verzögert wird oder ganz unterbleibt. Dies wird bevorzugt durch den gezielten Austausch von mindestens einer Aminosäure im Linker-Peptid des Wildtyps erreicht. Die Aminosäuresequenz des Linker-Peptids kann aber auch durch Einfügen oder Herausnehmen mindestens einer Aminosäure modifiziert sein. Unter dem Linker-Peptid des Wildtyps wird ein Linker-Peptid aus einer natürlicherweise vorkommenden Pro-Penicillinamidase verstanden, das in vivo vollständig von der Peptid-Einheit abgespalten wird. Ein Wildtyp-Linker-Peptid aus *A. faecalis* weist beispielsweise die Aminosäuresequenz der SEQ ID NO: 15 auf.

In einer besonders bevorzugten Ausführungsform wird mindestens eine Aminosäure im Linker-Peptid gegen Glycin oder Prolin ausgetauscht. Es ist aber auch möglich, Aminosäuren gegen andere, beispielsweise nicht natürlicherweise in Bakterien vorkommende, Aminosäuren auszutauschen, die eine Abspaltung des Linker-Peptids verhindern.

Im Falle der Penicillinamidase aus *A. faecalis* erfolgt ein Aminosäureaustausch besonders bevorzugt an den Positionen 206, 213, 219, 222, 231 (Numerierung bezogen auf die Pro-Penicillinamidase, SEQ ID NO: 1, SEQ ID NO: 2). Eine entsprechende Penicillinamidase umfaßt bevorzugt eine Aminosäuresequenz der SEQ ID NO: 16 bis SEQ ID NO: 36. Die erfindungsgemäß modifizierten Linker-Peptide werden insbesondere in vivo nicht vollständig von der A-Kette abgespalten. Der Aminosäureaustausch bewirkt, daß der vollständige Abbau des Linker-Peptids unterbunden oder zumindest stark verlangsamt wird, so daß der Linker oder ein Teil davon an der A-Kette kovalent gebunden bleibt. Eine Penicillinamidase mit derart modifizierter A-Kette weist gegenüber dem Wildtyp-Enzym, das kein Linker-Fragmente enthält, eine deutlich erhöhte Aktivität auf. Im Falle einer modifizierten Penicillinamidase mit einem Aminosäureaustausch an den Positionen 206, 213 und 219 ist die Aktivität beispielsweise um mehr als Doppelte gegenüber dem Wildtyp-Enzym erhöht. Die Erhöhung der Aktivität ist sowohl auf einen niedrigeren *K*ₘ-Wert (als Maß für die Substrataffinität) als auch einen erhöhten *k*_{cat}-Wert (als Maß für die Umsatzrate) des erfindungsgemäßen Enzyms zurückzuführen.

Die Erfindung betrifft auch eine modifizierte Pro-Penicillinamidase, bei der die Aminosäuresequenz des Linker-Peptids gegenüber dem Wildtyp in einer Weise modifiziert ist, daß eine vollständige Abspaltung des Linker-Peptids verzögert ist oder unterbleibt. Bevorzugt wird die Aminosäuresequenz dabei durch den Austausch mindestens einer Aminosäure modifiziert. Besonders bevorzugt wird die Aminosäure gegen Glycin oder Prolin ausgetauscht. Im Falle der Pro-Penicillinamidase aus A. faecalis erfolgt der Austausch besonders bevorzugt an den Positionen 206, 213, 219, 222, 231 (Numerierung bezogen auf die Pro-Penicillinamidase gemäß SEQ ID NO: 1). Eine entsprechende Pro-Penicillinamidase umfaßt bevorzugt eine Aminosäuresequenz, die von einer Nukleotidsequenz gemäß der SEQ ID NO: 3, SEQ ID NO: 5, SEQ ID NO: 7 oder SEQ ID NO: 9 kodiert wird, bzw. im wesentlichen der Aminosäuresequenz der SEQ ID NO: 4, SEQ ID NO: 6, SEQ ID NO: 8 oder SEQ ID NO: 10 entspricht. Weiter bevorzugt umfaßt die Pro-Penicillinamidase eine Aminosäuresequenz der SEQ ID NO: 16 bis SEQ ID NO: 36. Im Laufe der Reifung der Pro-Penicillinamidase wird das Linker-Peptid nicht vollständig abgespalten, sondern bleibt als Fragment an einer Peptid-Einheit der Penicillinamidase kovalent gebunden. Der Einsatz der erfindungsgemäßen Pro-Penicillinamidase ist insbesondere deshalb vorteilhaft, weil in vivo hieraus ohne weiteres eine entsprechend modifizierte Penicillinamidase hergestellt wird.

Weiter betrifft die Erfindung auch eine Nukleinsäure, die für eine erfindungsgemäße Penicillinamidase oder Pro-Penicillinamidase kodiert. Die Nukleinsäure kann eine DNA oder eine der DNA in ihrer Sequenz entsprechende RNA oder ein der DNA in ihrer Sequenz entsprechendes aus DNA- und RNA-Nukleotiden zusammengesetztes Mischmolekül sein. Die Nukleinsäure umfaßt dabei bevorzugt eine der Nukleotidsequenzen gemäß SEQ ID NO: 3, SEQ ID NO: 5, SEQ ID NO: 7 oder SEQ ID NO: 9. Durch den Einsatz der erfindungsgemäßen Nukleinsäuren wird es ermöglicht, mit Hilfe geeigneter molekularbiologischer Techniken Mikroorganismen herzustellen, die das modifizierte Gen für die Pro-Penicillinamidase exprimieren. Auf diese Weise ist eine effiziente und kostengünstige Produktion der Penicillinamidase auch in technischem Maßstab möglich.

Die Erfindung betrifft weiterhin ein Verfahren zur Veränderung der Aktivität eines Enzyms, wobei man aus einem Vorläufer-Enzym (Pro-Enzym) mit einem Linker-Peptid, das eine Aminosäurekette für eine erste Peptid-Einheit des Enzyms mit einer Aminosäurekette für eine zweite Peptid-Einheit des Enzyms verbindet, die Aminosäuresequenz des Linker-Peptids gegenüber der Aminosäuresequenz des Wildtyp-Linker-Peptids modifiziert und eine Abspaltung des Linker-Peptids von der ersten Peptid-Einheit verzögert oder verhindert.

Enzyme, die in vivo aus Vorläufer-Enzymen (Pro-Enzymen) durch Abspaltung von Aminosäuren oder Peptiden, beispielsweise Oligopeptiden, hergestellt werden, weisen in der Regel ihre endgültige Aktivität erst nach Abspaltung der Aminosäuren oder des Oligopeptids bzw. der Oligopeptide auf. Dabei kann, wie im Falle der Penicillinamidase, das endgültige Enzym eine geringere Aktivität aufweisen als ein Vorläufer-Molekül. Mit Hilfe des erfindungsgemäßen Verfahrens ist es nun möglich, gezielt die ansonsten stattfindende Abspaltung von Aminosäuren oder Oligopeptiden zu verhindern und solche Vorläufer-Enzyme praktisch in einem Abbau-Stadium zu "konservieren", in dem sie eine gegenüber dem endgültigen Enzym höhere Aktivität aufweisen. Das Verfahren ermöglicht es auch, beispielsweise ein Enzym mit geringerer Aktivität herzustellen, wenn dies gewünscht ist. Dies kann beispielsweise der Fall sein, wenn gekoppelte enzymatische Prozesse aufeinander abgestimmt werden sollen.

Häufig bevorzugen Enzyme, die Peptidbindungen spalten und auf diese Weise Peptide schneiden, bestimmte Schnittstellen, die beispielsweise durch eine oder mehrere charakteristische Aminosäuresequenzen gekennzeichnet sind. Das Spalten einer Peptidbindung an der Schnittstelle kann beispielsweise verhindert werden, indem entweder die Aminosäuresequenz an der Schnittstelle selbst derart modifiziert wird, daß das Enzym dort nicht mehr schneiden kann. Eine andere Möglichkeit besteht darin, daß die Aminosäuresequenz in der Nähe der Schnittstelle so modifiziert wird, daß das Enzym an dem Schneiden an der Schnittstelle gehindert wird. Dies kann z.B. durch sterische Hinderung oder Wegfall einer Erkennungsregion geschehen. Im Falle der vorliegenden Erfindung scheint es so zu sein, daß ein Aminosäureaustausch die Spaltung, die hier durch eine Hydrolyse der Peptidbindung erfolgt, an einer Schnittstelle in der Nähe der ausgetauschten Aminosäure verhindert. Dabei liegt die ausgetauschte Aminosäure zwischen der Schnittstelle und dem C-Terminus der Peptid-Einheit, an die das Linker-Peptid kovalent gebunden ist. Ein Aminosäureaustausch führt somit nicht notwendig dazu, daß eine weitere Abspaltung von Aminosäuren oder Oligopeptiden an der Stelle stoppt, an der sich die ausgetauschte Aminosäure befindet. Vielmehr stoppt die Hydrolyse nach bisherigen Kenntnissen an der zum C-Terminus des Linker-Peptids hin jeweils der ausgetauschten Aminosäure nächstliegenden Schnittstelle.

Bevorzugt wird die Aminosäuresequenz des Linker-Peptids durch den Austausch mindestens einer Aminosäure modifiziert. Besonders bevorzugt wird die Aminosäure durch die Aminosäure Glycin oder Prolin ersetzt.

In einer bevorzugten Ausführungsform des Verfahrens wird die Modifikation der Aminosäuresequenz des Linker-Peptids dadurch herbeigeführt, daß mindestens eine Mutation in den für das Linker-Peptid kodierenden Bereich des für das Enzym kodierenden Gens eingeführt wird. Bei der Expression des mutierten Gens wird ein entsprechend modifiziertes Vorläufer-Enzym (Pro-Enzym) synthetisiert, aus dem dann das endgültige Enzym hergestellt wird.

In einer weiteren Ausführungsform umfaßt das Verfahren darüber hinaus auch die Schritte:
a) Einführen des mutierten Gens in einen Vektor,
b) Einführen des Vektors in einen Mikroorganismus, der das veränderte Gen exprimiert, bevorzugt in *E. coli,*
c) Vermehren des Mikroorganismus,
e) Isolieren und Reinigen des Enzyms.

Bevorzugt weist das Enzym eine gegenüber dem Wildtyp erhöhte Aktivität auf. Dies ist besonders wichtig für Enzyme, die im industriellen Maßstab eingesetzt werden sollen. Jede Aktivitätserhöhung gegenüber bisher bekannten Enzymen erlaubt eine schnellere und kostengünstigere Herstellung des mit Hilfe des Enzyms gefertigten Produkts.

Besonders bevorzugt handelt es sich bei dem hergestellten Enzym um die Penicillinamidase (EC 3.5.1.11). Weiter bevorzugt handelt es sich bei dem Enzym um die Penicillinamidase aus *A. faecalis.*

Weiterhin betrifft die Erfindung auch ein Verfahren zur Herstellung von 6-Aminopenicillansäure (6-APA) und/oder 7-Aminodesacetoxycephalosporansäure (7-ADCA), wobei eine erfindungsgemäße Penicillinamidase oder Pro-Penicillinamidase eingesetzt wird. Bevorzugt ist die Penicillinamidase immobilisiert.

Die Erfindung betrifft auch eine Zusammensetzung, die mindestens ein Oligopeptid enthält, das eine der Aminosäuresequenzen der SEQ ID 16 bis SEQ ID 36 aufweist sowie die Verwendung der Zusammensetzung zur Erhöhung der Aktivität einer Penicillinamidase, bevorzugt der Penicillinamidase aus *A. faecalis.* Es hat sich gezeigt, daß eine Aktivitätssteigerung bei der Penicillinamidase bereits durch die Gegenwart von Linker-Peptid-Fragmenten, beispielsweise in einer gemeinsamen Lösung, bewirkt werden kann, ohne daß eine kovalente Bindung an das Enzym vorliegen muß. Die erfindungsgemäße Zusammensetzung enthält daher mindestens ein Linker-Peptid-Fragment und kann beispielsweise verwendet werden, um die Aktivität des Wildtyp-Enzyms zu erhöhen. Die Linker-Peptid-Fragmente können beispielsweise mit Hilfe üblicher Techniken synthetisch hergestellt werden.

Darüber hinaus betrifft die Erfindung auch die Verwendung einer Zusammensetzung entsprechender Fragmente des Wildtyp-Linker-Peptids (SEQ ID NO: 15) zur Erhöhung der Aktivität einer Penicillinamidase, bevorzugt der Penicillinamidase aus *A. faecalis,* wobei die Zusammensetzung mindestens ein Oligopeptid enthält, das eine der Aminosäuresequenzen der SEQ ID 15, SEQ ID 37 bis SEQ ID 41 aufweist. Die Linker-Peptid-Fragmente können beispielsweise mit Hilfe üblicher Techniken synthetisch hergestellt werden.

Des weiteren betrifft die Erfindung auch Vektoren oder Wirtszellen, die eine erfindungsgemäße Nukleinsäure enthalten. Die Nukleinsäure kann eine DNA oder eine der DNA in ihrer Sequenz entsprechende RNA oder ein der DNA in ihrer Sequenz entsprechendes aus DNA- und RNA-Nukleotiden zusammengesetztes Mischmolekül sein. Vektoren, die zur Einführung von Nukleotidsequenzen in Wirtszellen geeignet sind, beispielsweise Viren, Bakteriophagen, Cosmide, Plasmide, künstliche Hefechromosomen, T-DNA, Transposons, Insertionssequenzen usw., sind dem Fachmann auf dem Gebiet molekularer Klonierungstechniken bekannt.

Die Erfindung wird im folgenden an Hand von Ausführungsbeispielen und der Figuren näher beschrieben. Es zeigt:
Fig. 1. Konstruktion des Plasmids pPAAF. Die verwendeten Primer sind mit Großbuchstaben dargestellt, das *A.*-*faecalis*-pac-Gen in Kleinbuchstaben und eingerahmt. Die unterstrichenen Symbole kennzeichnen die bei der Klonierung ausgetauschten Basen.
Fig. 2. In-vitro-Einfluß von chemisch synthetisierten Teilen des A.-faecalis-Linker-Peptids auf A.-faecalis-PA-Aktivität. Der Startpunkt gibt die Aktivität der *A.-faecalis*-Penicillinamidase ohne Zugabe von Oligopeptiden wieder. Symbole: 11mer (●), 20mer (▲), 29mer (○).

### Ausführungsbeispiele

Für vergleichende Untersuchungen über die Substrat- und Stereoselektivität von Penicillinamidasen (Kasche, V. et al., Biotechnol. Lett. 18, 455-460, 1996; Ignatova, Z. et al., Biotechnol. Lett. 20, 977-982, 1998; B. Galunsky et al., Chem. Monthly 131, 623-632, 2000) aus verschieden Organismen wurde u.a. das Penicillinamidase-Gen aus *A. faecalis* kloniert und in *E. coli* exprimiert. Bei der Aufarbeitung des Enzyms aus dem Homogenat wurde dieses vor der Chromatographie aufkonzentriert. Dabei wurde unerwartet gefunden, daß die Enzymaktivität des konzentrierten Homogenates durch Zugabe des abgetrennten Filtrates bis zu einem Faktor von 2,5 gesteigert werden konnte. Ausgehend von diesen Beobachtungen wurde das konzentrierte Enzym mit verschiedenen synthetischen Oligopeptiden inkubiert, die den in der sequentiellen Prozessierung des Linkers entstehenden unterschiedlich langen Fragmenten entsprachen. Dabei wurde eine Aktivitätserhöhung von bis zu einem Faktor von 2,3 im Vergleich zum gereinigten Wildtyp-Enzym aus *A. faecalis* beobachtet. Im technischen Maßstab ist eine Aktivierung von Enzymen durch Zugabe von Peptiden vergleichsweise schwer zu realisieren. Wenn jedoch die Prozessierung der Penicillinamidase aus A. faecalis durch einen mit den üblichen molekularbiologischen Methoden durchgeführten Aminosäureaustausch so geändert wird, daß das aktivierende Linker-Peptid an dem C-Ende der A-Kette kovalent gebunden bleibt, kann dieses mutierte Enzym auch für die oben genannten Zwecke eingesetzt werden. Durch die Erhöhung der Enzymaktivität kann die Enzymmenge, die für Hydrolyse einer bestimmten Menge Penicillin G oder 3-Desacetoxycephalosporin in einer gegebenen zeit benötigt wird, um bis zu einem Faktor von 2,3 reduziert werden.

Nach der Expression eines für die oben genannten Zwecke gezielt mutierten A.-faecalis-Gens, das für eine Pro-Penicillinamidase mit im Linker veränderter Primärstruktur kodiert, wird durch proteolytische Aktivierung des Vorläufer-Moleküls eine modifizierte Penicillinamidase mit verlängerter A-Kette gebildet, die eine höhere spezifische Aktivität gegenüber dem Wildtyp-Enzym aufweist.

### Klonierung und Identifizierung des Penicillinamidase-Gens

Alle Klonierungstechniken wurden nach Standardprotokollen (Maniatis et al., 1982 and 1989, Molecular Cloning, Cold Spring Harbor Laboratory) durchgeführt. Alle Enzyme für die DNA-Manipulationen wurden nach den Anleitungen der entsprechenden öffentlich zugänglichen Quellen eingesetzt (DSMZ, Deutschland oder Morales, V. M. et al., Gene 97, 39-47, 1991). Der site-spezifische Austausch von Basenpaaren in der DNA-Sequenz erfolgte mit dem "QuickChange Mutagenesis Kit" (Stratagene). Alle durchgeführten Mutationen wurden durch DNA-Sequenzierung überprüft (SeqLab, Germany).

### Kultivierungsmedien

Für alle gentechnischen Manipulationen wurden die Wirtszellen in flüssigen LB-Medium aerob kultiviert oder auf festes LB-Agar-Medium (15 g/l Agar-agar), versetzt mit einem entsprechenden Antibiotikum, ausplatiert (Maniatis et al., 1982 and 1989, Molecular Cloning, Cold Spring Harbor Laboratory).

### LB Medium:

10 g/l Trypton; 10 g/l NaCl; 5 g/l Hefeextrakt (pH 7,5)

### M9 Medium:

6 g/l Na₂HPO₄; 3 g/l KH₂PO₄; 0.5 g/l NaCl; 1 g/l NH₄Cl; 2 mM MgS0₄; 0,1 mM CaCl₂; 2,5 g/l Glucose (pH 7,4)

### Klonierung des A.-faecalis-pac-Gens

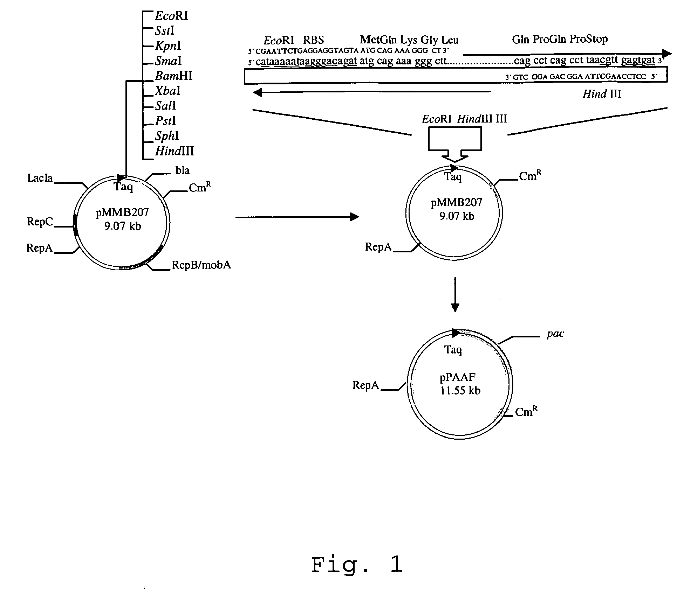

Das A.-faecalis-pac-Gen wurde von der chromosomalen DNA von *A. faecalis* (ATCC 19018) als Template mit folgenden Primern 5'-CGAATTCTGAGGAGGTAGTAATGCAGAAAGGGCT-3' und 5'-CCTCCAAGCTTAAGGCAGAGGCTG-3' amplifiziert. Das gereinigte PCR-Fragment (2360 bp), welches das pac-Gen mit den entsprechenden RBS (ribosomal binding sites) und Shine-Dalgarno-Sequenzen enthielt, wurde mit EcoRI und Hind III geschnitten und in den Polylinker des Plasmids pMMB207 (9,07 kb) ligiert. Das resultierende Plasmid pPAAF (Fig. 1) wurde in E.-coli-DH5a-Wirtszellen transformiert und auf mit dem entsprechenden Selektionsmarker (Chloramphenicol) versehene LB-Agar-Platten mit einem Nitrocellulosefilter ausplatiert. Die pac-haltigen Klone wurden phänotypisch auf Penicillinamidaseaktivität mit dem chromogenen Substrat 6-nitro-3-phenylacetamido Benzoesäure, NIPAB, (Zhang, Q. et al., Anal. Biochem. 156, 413-416, 1986) selektiert.

### Mutationen im Linker-Peptid der A.-faecalis-Penicillinamidase

Von einem PA-positiven Klon wurde die Plasmid-DNA isoliert und als Template für den site-spezifischen Austausch von Aminosäuren verwendet. Nach dem DpnI-Verdau der parentalen DNA wurden die mit Hilfe von PCR-amplifizierten Plasmide in E. *coli* DH5α transformiert. Die eingeführten Mutationen (Tabelle 1) wurden durch DNA-Sequenzierung überprüft (SeqLab, Germany).

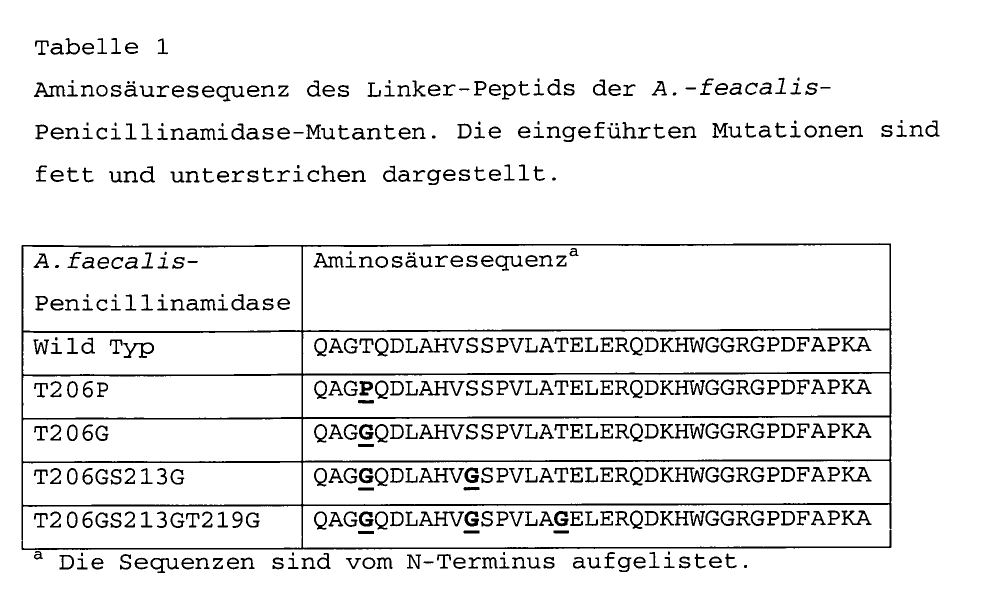

### Expression und Reinigung der A.-faecalis-Penicillinamidasen mit Mutationen im Linker-Peptid

Sowohl das pPAAF Plasmid als auch die im pac-Gen mutationshaltigen Plasmide wurden in E.-coli-BL21(DE3)-Wirtszellen transformiert und bei 28 °C in M9-Medium mit 2,5 g/l Glucose kultiviert. Induktion erfolgte mit 0,5 mM IPTG. Für die Beobachtung der Penicillinamidasesynthese während der Kultivierung wurden die stündlich entnommenen Proben homogenisiert (Branson Ultraschall Desintegrator W-450, 50% duty cycle, output 3, Heinemann, Deutschland) und auf Penicillinamidaseaktivität mit dem chromogenen Substrat NIPAB getestet (Kasche, V. et al., Ann. N. Y. Acad. Sci. 501, 97-102, 1987). Die spezifische Aktivitäten sind als eine Absorbanzänderung bei 380 nm pro Minute per Probenabsorbanz bei 280 nm (ΔA₃₈₀/(min•A₂₈₀)) dargestellt. Die Zellen wurden am Anfang der stationären Phase abgeerntet. Für die Isolierung der periplasmatischen Fraktion wurden die Zellen mit mildem osmotischen Schock (Rodrigues, M. et al., Process Biochem. 27, 217-223, 1992) aufgeschlossen. Die *A.-faecalis*-Penicillinamidase und die Penicillinamidasen mit Mutationen im Linker-Peptid wurden mit Anionaustausch-Chromatographie aufgereinigt (Ignatova, Z. et al., Biotechnol. Lett. 20, 977-982, 1998) und die gesammelten penicillinamidase-haltigen Fraktionen anschließend nach dem Pufferaustausch (mit destilliertem Wasser) 10-fach aufkonzentriert (Amicon, 10K). Die Enzymhomogenität wurde mit Hilfe von SDS-PAGE (Laemmli, U.K., Nature 227, 680-685, 1970) und IEF (Serva, Deutschland) überprüft. Die Konzentration der A.-faecalis-Penicillinamidase und der Mutanten wurde mittels "Active-site"-Titration mit PMSF ermittelt (Svedas, V.K. et al., Bioorg. Khim. 3, 546-553, 1977).

### Vergleich der Immobilisierungsausbeute von Wildtyp-A.faecalis-Penicillinamidase und deren T206G- und T206GS213G-Mutanten

Es wurden Experimente zur Immobilisierung von Wildtyp *A.faecalis*-Penicillinamidase und T206G- und T206GS213G-Mutanten auf BrCN-Sepharose (Pharmacia) durchgeführt. Jeweils 2 ml von den abzentrifugierten und konzentrierten (10 K, Amicon) Zellhomogenaten (siehe oben) von Wildtyp, T206G- und T206GS213G-Mutanten wurden aliquotiert, pH auf 8,2 und die Ionenstärke auf 0,5 (mit NaCl) eingestellt. Daraufhin wurden diese 2 ml mit 1 ml gequollener BrCN-Sepharose 2 Stunden bei Raumtemperatur inkubiert (geschüttelt). Nach der Filtrierung und dem alternativen Waschen mit 0,5M-NaCl-haltigen 0.1M Acetatpuffer (pH 4,0) und 0,1 M Phosphatpuffer (pH 7,5) wurden die restlichen aktiven Gruppen an den Trägern mit 1 M Ethanolamin (pH 8,0) blockiert. Von den mit NIPAB gemessenen Aktivitäten in den Überständen und den Waschungen wurden die Aktivitätsbilanzen der Immobilisierung erstellt. Zum Schluß wurden die Aktivitäten der immobilisierten Penicillinamidasen direkt in Suspension gemessen (B. Galunsky et al., Anal. Biochem. 221, 213-214, 1994). Die Ergebnisse sind in Tabelle 2 dargestellt.

Bei der Immobilisierung der Wildtyp-*A.-faecalis*-Penicillinamidase aus Zellhomogenat wurde eine Reduktion der Aktivität beobachtet, die wahrscheinlich auf das Auswaschen der Linkerfragmente zurückzuführen ist. Das Blockieren der weiteren Prozessierung des Linkers bei der T206G-Mutante und T206GS213G-Mutante hatte dagegen keine Aktivitätsverluste bei der Immobilisierung zur Folge.

**Tabelle 2**

| Vergleich der Immobilisierungsausbeute von Wildtyp-A.- faecalis-Penicillinamidase und T206G- und T206GS213G-Mutanten an BrCN-Sepharose^{a} | | | | | |
|---|---|---|---|---|---|
| Enzyme | Relative Einheiten^{b} zugegeben für Immobilisierung an 1 ml Träger | Gesamtmenge der relativen Einheiten in Überstand und Waschungen | Erwartete relative Einheiten pro ml Träger | Direkt bestimmte relative Einheiten pro ml Träger^{c} | Aktivitätsverlust durch die Immobilisierung (%) |
| Wildtyp | 4,60 | 1,20 | 3,40 | 1,20 | 65 |
| T206G-Mutante | 1,10 | 0,60 | 0,50 | 0,48 | 4 |
| T206GS213G-Mutante | 1,70 | 0,34 | 1,36 | 1,33 | 2 |

| | | | | | |
|---|---|---|---|---|---|
| ^{a} Als Kontrolle zu jedem der zur Immobilisierung angesetzten Homogenate diente ein Reaktionsansatz ohne Träger. ^{b} Die Aktivität wurde im 1 ml Endvolumen mit 125 µM NIPAB im Phospatpuffer pH 7,5 bei 25 °C mit 25 µl passend verdünnter PA-Lösung oder Suspension von dem immmobilisierten Enzym gemessen. Die Absorbanzänderung bei 380 nm ergibt die relativen Units pro ml PA-Lösung oder pro ml immobilisiertes Enzym. | | | | | |
| ^{c} Diese Ergebnisse wurden auch mit "Active-site"-Titration mit PMSF bestätigt (Svedas, V.K. et al., Bioorg. Khim. 3, 546-553, 1977). | | | | | |

### In-vitro-Einfluß der chemisch synthetisierten Linker-Peptid-Fragmente auf die A.-faecalis-Penicillinamidaseaktivität

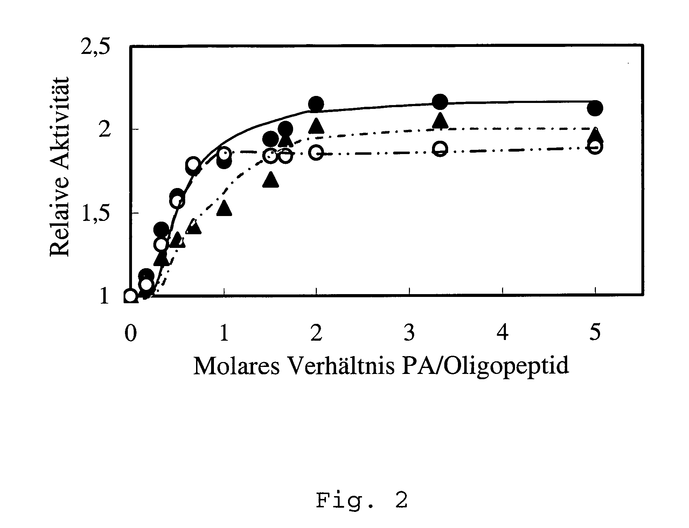

Unterschiedliche Konzentrationen (0-100 nM) der chemisch synthetisierten Fragmente vom *A.-faecalis*-Linker-Peptid (ARK Scientific, Deutschland), s. Tabelle 3, wurden in-vitro mit gereinigter *A.-faecalis*-Penicillinamidase (15 nM) 15 min bei 25 °C in Phosphatpuffer pH 7.5 *I*=0.2 präinkubiert und anschließend die Penicillinamidaseaktivität mit NIPAB gemessen (Fig. 2).

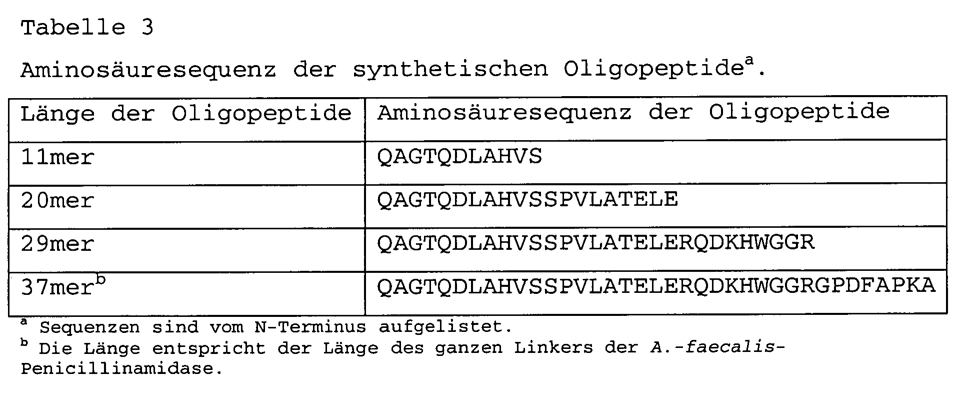

In-vitro-Inkubation von A.-faecalis-Penicillinamidase mit den Oligopeptiden, die der Sequenz der unterschiedlich langen Fragmente ihres Linker-Peptids entsprachen, führte zu einer Erhöhung der Aktivität bis zu einem Faktor von 2,3 (Fig. 2). Der stärkste Einfluß wurde mit dem kürzesten Oligopeptid (11mer) beobachtet. Seine aktivierende Wirkung wies eine Konzentrationsabhängigkeit auf. Die Erhöhung der Konzentration vom 29mer Oligopeptid über das stoichiometrische Verhältnis zur Penicillinamidase hatte keinen Effekt. Im Fall des 11mer Oligopeptids dagegen stieg die Penicillinamidaseaktivität bis zu einem Verhältnis von 1:2 (Penicillinamidase : 11mer).

### Vergleich der spezifischen Aktivitäten von Wildtyp-A.faecalis-Penicillinamidase und A.-faecalis-Penicillinamidase-Mutanten

Die spezifischen Aktivitäten von *A.-faecalis*-Penicillinamidase und *A.*-*faecalis*-Penicillinamidase-Mutanten wurden mit dem chromogenen Substrat NIPAB und Benzylpenicillin getestet. Gleiche Konzentrationen (bestimmt mittels "Active-site"-Titration mit PMSF) der gereinigten Enzyme wurden angesetzt und die Aktivität bei 25 °C in Phosphatpuffer (pH 7,5, *I*=0,2) bestimmt (Tabelle 4). Aus den in Tabelle 4 zusammengefaßten Ergebnissen kann geschlossen werden, daß die Blockierung der Spaltung im Linker-Peptid bei der Aminosäure 206 (T206G-Mutante) zu einer 1,6-fachen Erhöhung der spezifischen Aktivität führt. Darüber hinaus führt das Einfügen zusätzlicher Mutationen im Linker-Peptid zu einem 1,9-fachen Aktivitätsanstieg bei der T206GS213G-Mutante und 2,3-fachen Aktivitätserhöhung bei der T206GS213GT219G-Mutante im Vergleich zur Wildtyp-*A.-faecalis*-Penicillinamidase.

**Tabelle 4**

| Spezifische Aktivität der Wildtyp-*A.-faecalis*-Penicillin-amidase und *A.-faecalis*-Penicillinamidase-Mutanten. | |
|---|---|
| Enzyme | Spezifische Aktivität^{a}, ΔA₃₈₀/ (min•A₂₈₀) |
| Wildtyp-*A.-faecalis*-PA | 2,0 |
| T206G-Mutante | 3,2 |
| T206GS213G-Mutante | 3,7 |
| T206GS213GT219G-Mutante | 4,5 |

| | |
|---|---|
| ^{a} Definition s. oben | |

Die *K*ₘ- und *k*_{cat}-Werte für die Benzylpenicillinhydrolyse wurden von Anfangsgeschwindigkeiten bei unterschiedlichen Substratkonzentrationen (zwischen 5 and 80 µM) mittels HPLC analysiert. Die *K*ₘ-Werte waren für die Wildtyp-*A.-faecalis*-Penicillinamidase und die T206G-Mutante vergleichbar (s. Tabelle 5). Der errechnete um 50% Prozent erhöhte Wert der Spezifizitätskonstante (*k*_{cat}/*K*ₘ) bei der T206G-Mutante ist Folge des höheren *k*_{cat}-Wertes. Die zusätzliche zweite (T206GS213G-Mutante) und dritte (T206GS213GT219G-Mutante) Mutation im Linker-Peptid bewirkte infolge eines Anstiegs von *k*_{cat} und einer Verringerung von *K*ₘ eine 2-fache, bzw. 3,1-fache Erhöhung der Spezifizitätskonstante (*k*_{cat}/*K*ₘ) .

**Tabelle 5**

| | | | |
|---|---|---|---|
| Kinetische Parameter^{a} von Wildtyp-A.-faecalis-Penicillinamidase und A.-faecalis-Penicillinamidase-Mutanten für die Hydrolyse von Benzylpenicillin bei 25 °C und pH 7,5 (Phosphatpuffer, I=0,2). | | | |

| Enzyme | Kinetische Konstanten | | |
|---|---|---|---|
| | *K*_{*m*} (M) | *k*_{*cat*} (s⁻¹) | *k*_{*cat*}/*K*_{*m*} (M⁻¹s⁻¹) |
| Wildtyp-*A.-faecalis*-PA^{b} | 8 x 10⁻⁶ | 80 | 1,0 x 10⁷ |
| T206G-Mutante | 8 x 10⁻⁶ | 120 | 1,5 x 10⁷ |
| T206GS213G-Mutante | 7 x 10⁻⁶ | 140 | 2,0 x 10⁷ |
| T206GS213GT219G-Mutante | 6 x 10⁻⁶ | 190 | 3,1 x 10⁷ |

| | | | |
|---|---|---|---|
| ^{a} Der experimentelle Fehler ist kleiner als 15%. | | | |
| ^{b}Ignatova, Z. et al., Biotechnol. Lett. 20, 977-982, 1998. | | | |

## Patentansprüche

1. Penicillinamidase mit mindestens einer Peptid-Einheit, **dadurch gekennzeichnet, daß** an die Peptid-Einheit ein Linker-Peptid oder ein Teil eines Linker-Peptids kovalent gebunden ist, wodurch sich die Aktivität der Penicillinamidase gegenüber einer Penicillinamidase, die die gleiche Peptid-Einheit ohne das gebundene Linker-Peptid oder ohne den gebundenen Teil des Linker-Peptids enthält, erhöht.

2. Penicillinamidase nach Anspruch 1, **dadurch gekennzeichnet, daß** das Linker-Peptid oder ein Teil des Linker-Peptids von einem Vorläufer-Enzym (Pro-Enzym) stammt, bei dem das Linker-Peptid in vivo die Aminosäurekette der Peptid-Einheit mit der Aminosäurekette einer weiteren Peptid-Einheit verbindet.

3. Penicillinamidase nach Anspruch 1 oder 2, **dadurch gekennzeichnet, daß** das Linker-Peptid eine Aminosäuresequenz aufweist, die derart gegenüber der Aminosäuresequenz des Wildtyp-Linker-Peptids modifiziert ist, daß eine Abspaltung des Linker-Peptids verzögert oder unterbunden ist.

4. Penicillinamidase nach Anspruch 3, **dadurch gekennzeichnet, daß** die Aminosäuresequenz des Linker-Peptids durch den Austausch mindestens einer Aminosäure modifiziert ist.

5. Penicillinamidase nach Anspruch 4, **dadurch gekennzeichnet, daß** die Aminosäure gegen Glycin oder Prolin ausgetauscht ist.

6. Penicillinamidase nach einem der Ansprüche 1 bis 5, umfassend eine Aminosäuresequenz der SEQ ID NO: 16 bis SEQ ID NO: 36.

7. Pro-Penicillinamidase mit einem Linker-Peptid, das die Aminosäurekette für eine erste Peptid-Einheit einer Penicillin-amidase mit der Aminosäurekette für eine zweite Peptid-Einheit der Penicillinamidase verbindet, **dadurch gekennzeichnet, daß** das Linker-Peptid eine Aminosäuresequenz aufweist, die geeignet ist, eine Abspaltung des Linker-Peptids von der Aminosäurekette der ersten Peptid-Einheit zu verzögern oder zu unterbinden.

8. Pro-Penicillinamidase nach Anspruch 7, **dadurch gekennzeichnet, daß** die Aminosäuresequenz des Linker-Peptids durch den Austausch mindestens einer Aminosäure modifiziert ist.

9. Pro-Penicillinamidase nach Anspruch 8, **dadurch gekennzeichnet, daß** die Aminosäure gegen Glycin oder Prolin ausgetauscht ist.

10. Pro-Penicillinamidase nach einem der Ansprüche 7 bis 9, umfassend eine Aminosäuresequenz, die
a) von einer Nukleotidsequenz gemäß der SEQ ID NO: 3, SEQ ID NO: 5, SEQ ID NO: 7 oder SEQ ID NO: 9 kodiert wird, oder
b) im wesentlichen einer Aminosäuresequenz der SEQ ID NO: 4, SEQ ID NO: 6, SEQ ID NO: 8 oder SEQ ID NO: 10 entspricht.

11. Pro-Penicillinamidase nach einem der Ansprüche 7 bis 10, umfassend eine Aminosäuresequenz, die einer Aminosäuresequenz der SEQ ID NO: 16 bis SEQ ID NO: 36 entspricht.

12. Nukleinsäure, die für eine Penicillinamidase gemäß einem der Ansprüche 1 bis 6 kodiert.

13. Nukleinsäure, die für eine Pro-Penicillinamidase gemäß einem der Ansprüche 7 bis 11 kodiert.

14. Nukleinsäure nach Anspruch 13, **dadurch gekennzeichnet, daß** die Nukleinsäure eine der Nukleotidsequenzen gemäß SEQ ID NO: 3, SEQ ID NO: 5, SEQ ID NO: 7 oder SEQ ID NO: 9 umfaßt.

15. Verfahren zur Veränderung der Aktivität eines Enzyms, **dadurch gekennzeichnet, daß** man bei einem Vorläufer-Enzym (Pro-Enzym) mit einem Linker-Peptid, das eine Aminosäurekette für eine erste Peptid-Einheit des Enzyms mit einer Aminosäurekette für eine zweite Peptid-Einheit des Enzyms verbindet, die Aminosäuresequenz des Linker-Peptids gegenüber der Aminosäuresequenz des Wildtyp-Linker-Peptids derart modifiziert, daß eine vollständige Abspaltung des Linker-Peptids von der ersten Peptid-Einheit verzögert oder verhindert wird.

16. Verfahren nach Anspruch 15, **dadurch gekennzeichnet, daß** man die Aminosäuresequenz des Linker-Peptids durch den Austausch mindestens einer Aminosäure modifiziert.

17. Verfahren nach Anspruch 16, **dadurch gekennzeichnet, daß** man die Aminosäure gegen Glycin oder Prolin austauscht.

18. Verfahren nach einem der Ansprüche 15 bis 17, **dadurch gekennzeichnet, daß** man die Modifikation der Aminosäuresequenz des Linker-Peptids durch Einführen mindestens einer Mutation in den für das Linker-Peptid kodierenden Bereich eines Gens herbeiführt.

19. Verfahren nach Anspruch 18, umfassend die weiteren Schritte:
a) Einführen des mutierten Gens in einen Vektor,
b) Einführen des Vektors in einen Mikroorganismus, der das veränderte Gen exprimiert, bevorzugt in *E. coli,*
c) Vermehren des Mikroorganismus,
d) Isolieren und Reinigen des Enzyms.

20. Verfahren nach einem der Ansprüche 15 bis 19, **dadurch gekennzeichnet, daß** das Enzym eine gegenüber dem Wildtyp erhöhte Aktivität aufweist.

21. Verfahren nach einem der Ansprüche 15 bis 20, **dadurch gekennzeichnet, daß** das Enzym Penicillinamidase (EC 3.5.1.11) ist.

22. Verfahren nach Anspruch 21, **dadurch gekennzeichnet, daß** die Penicillinamidase aus *Alcaligenes faecalis* ist.

23. Verfahren zur Herstellung von 6-Aminopenicillansäure (6-APA) und/oder 7-Aminodesacetoxycephalosporansäure (7-ADCA), **dadurch gekennzeichnet, daß** eine Penicillinamidase nach einem der Ansprüche 1 bis 6 oder eine Pro-Penicillinamidase nach einem der Ansprüche 7 bis 11 eingesetzt wird.

24. Verfahren nach Anspruch 23, **dadurch gekennzeichnet, daß** die Penicillinamidase immobilisiert ist.

25. Zusammensetzung, enthaltend mindestens ein Oligopeptid, das eine der Aminosäuresequenzen der SEQ ID 16 bis SEQ ID 36 aufweist.

26. Verwendung der Zusammensetzung nach Anspruch 25 zur Erhöhung der Aktivität einer Penicillinamidase.

27. Verwendung einer Zusammensetzung, enthaltend mindestens ein Oligopeptid, das eine der Aminosäuresequenzen der SEQ ID 15, SEQ ID 37 bis SEQ ID 41 aufweist, zur Erhöhung der Aktivität einer Penicillinamidase.

28. Verwendung nach Anspruch 26 oder 27, **dadurch gekennzeichnet, daß** die Penicillinamidase aus *Alcaligenes faecalis* ist.

29. Vektor, enthaltend mindestens eine Nukleinsäuresequenz gemäß einem der Ansprüche 12 bis 14.

30. Wirtszelle, enthaltend mindestens eine Nukleinsäuresequenz gemäß einem der Ansprüche 12 bis 14.


SEQUENZPROTOKOLL

<110>  TUHH Technologie GmbH

       Technische Universität Hamburg-Harburg

<120>  Gentechnisch veränderte Penicillinamidase und Verfahren zu ihrer
Herstellung

<130>  PAT 0888 EP

<150>  DE 10251747.9

<151>  2002-11-05

<160>  41

<170>  PatentIn version 3.1

<210>  1

<211>  2373

<212>  DNA

<213>  Alcaligenes faecalis

<220>

<221>  CDS

<222>  (1)..(2373)

<223>  Nukleinsäure kodierend für Pro-Penicillinamidase

<400>  1
caa gtg cag tcg gta gag gtg atg cgg gac agt tat ggc gtg ccg cac        48
Gln Val Gln Ser Val Glu Val Met Arg Asp Ser Tyr Gly Val Pro His
1               5                   10                  15

gtc ttt gcc gac agc cac tat ggc ttg tat tac ggc tat ggt tat gcg        96
Val Phe Ala Asp Ser His Tyr Gly Leu Tyr Tyr Gly Tyr Gly Tyr Ala
            20                  25                  30

gtc gcc caa gac cgt ctg ttc cag atg gac atg gcg cgt cgc tcc ttt       144
Val Ala Gln Asp Arg Leu Phe Gln Met Asp Met Ala Arg Arg Ser Phe
        35                  40                  45

gtc ggc aca acc gcc gcc gtc tta ggc cct ggt gag caa gat gcc tac 192
Val Gly Thr Thr Ala Ala Val Leu Gly Pro Gly Glu Gln Asp Ala Tyr
    50                  55                  60

gtc aag tac gac atg cag gtg cgg cag aac ttc acc ccg gct tcc ata 240
Val Lys Tyr Asp Met Gln Val Arg Gln Asn Phe Thr Pro Ala Ser Ile
65                  70                  75                  80

cag cgg cag atc gcg gcc ttg tcc aag gat gag cgc gat att ttt cgt 288
Gln Arg Gln Ile Ala Ala Leu Ser Lys Asp Glu Arg Asp Ile Phe Arg
                85                  90                  95

ggc tat gcc gat ggc tat aac gcc tat ctg gag cag gtg cgg cgt cgc 336
Gly Tyr Ala Asp Gly Tyr Asn Ala Tyr Leu Glu Gln Val Arg Arg Arg
            100                 105                 110

cct gag ttg ctg ccc aaa gaa tat gtg gat ttt gat ttc cag ccc gag 384
Pro Glu Leu Leu Pro Lys Glu Tyr Val Asp Phe Asp Phe Gln Pro Glu
        115                 120                 125

ccg ctg acc gac ttt gat gtg gtc atg atc tgg gtg ggc tcc atg gcc 432
Pro Leu Thr Asp Phe Asp Val Val Met Ile Trp Val Gly Ser Met Ala
    130                 135                 140

aat cgc ttc tcc gac acg aat ctg gaa gtg acg gca ctg gcc atg cgt 480
Asn Arg Phe Ser Asp Thr Asn Leu Glu Val Thr Ala Leu Ala Met Arg
145                 150                 155                 160

cag tct ctg gag aaa cag cac ggc ccg gaa cga ggc cgt gcc ttg ttt 528
Gln Ser Leu Glu Lys Gln His Gly Pro Glu Arg Gly Arg Ala Leu Phe
                165                 170                 175

gat gag ctg ctg tgg atc aat gac aca aca gct ccc act acg gtt ccg 576
Asp Glu Leu Leu Trp Ile Asn Asp Thr Thr Ala Pro Thr Thr Val Pro
            180                 185                 190

gcc ccc gct gcc gag cac aag ccg cag gca caa gca ggg acg cag gat 624
Ala Pro Ala Ala Glu His Lys Pro Gln Ala Gln Ala Gly Thr Gln Asp
        195                 200                 205

ctg gct cat gtt tcc tcg cca gta ctg gct acc gag cta gag cgc cag 672
Leu Ala His Val Ser Ser Pro Val Leu Ala Thr Glu Leu Glu Arg Gln
    210                 215                 220

gac aag cac tgg ggc ggc cgt ggc ccg gac ttc gcg ccc aag gct agc 720
Asp Lys His Trp Gly Gly Arg Gly Pro Asp Phe Ala Pro Lys Ala Ser
225                 230                 235                 240

aac ctg tgg agc act cgc ccc gag cga gtg cag gag ggc tcg acc gta 768
Asn Leu Trp Ser Thr Arg Pro Glu Arg Val Gln Glu Gly Ser Thr Val
                245                 250                 255

ctg atc aac ggc cca cag ttt ggc tgg tac aac ccg gcc tac acc tat 816
Leu Ile Asn Gly Pro Gln Phe Gly Trp Tyr Asn Pro Ala Tyr Thr Tyr
            260                 265                 270

ggc att ggc ttg cat ggc gcc ggc ttc gat gtg gtg ggt aat acg cct 864
Gly Ile Gly Leu His Gly Ala Gly Phe Asp Val Val Gly Asn Thr Pro

        275                 280                 285

ttt gcc tat ccg atc gta ctg ttt ggc acc aat agc gag att gcc tgg 912
Phe Ala Tyr Pro Ile Val Leu Phe Gly Thr Asn Ser Glu Ile Ala Trp
    290                 295                 300

ggg gcg act gct ggc ccg caa gat gtg gtg gac ata tat cag gaa aaa 960
Gly Ala Thr Ala Gly Pro Gln Asp Val Val Asp Ile Tyr Gln Glu Lys
305                 310                 315                 320

ttg aac ccc tcg cgt gcc gat cag tac tgg ttc aac aat gcc tgg cgc 1008
Leu Asn Pro Ser Arg Ala Asp Gln Tyr Trp Phe Asn Asn Ala Trp Arg
                325                 330                 335

acg atg gag cag cgc aag gaa cgt atc cag gta cgc ggt cag gct gat 1056
Thr Met Glu Gln Arg Lys Glu Arg Ile Gln Val Arg Gly Gln Ala Asp
            340                 345                 350

cgg gaa atg acg atc tgg cgc acc gtg cac ggc cct gtg atg cag ttt 1104
Arg Glu Met Thr Ile Trp Arg Thr Val His Gly Pro Val Met Gln Phe
        355                 360                 365

gat tac gat cag ggc gcg gcg tac agc aag aaa cgc agc tgg gat ggc 1152
Asp Tyr Asp Gln Gly Ala Ala Tyr Ser Lys Lys Arg Ser Trp Asp Gly
    370                 375                 380

tat gag gtg cag tcc ttg cta gcc tgg ttg aac gtg gcc aag gcc cgc 1200
Tyr Glu Val Gln Ser Leu Leu Ala Trp Leu Asn Val Ala Lys Ala Arg
385                 390                 395                 400

aac tgg acg gag ttt ctg gat caa gcc agc aag atg gcg att tcg atc 1248
Asn Trp Thr Glu Phe Leu Asp Gln Ala Ser Lys Met Ala Ile Ser Ile
                405                 410                 415

aac tgg tac tac gcc gac aag cac ggc aat att ggt tat gtc tcg ccg 1296
Asn Trp Tyr Tyr Ala Asp Lys His Gly Asn Ile Gly Tyr Val Ser Pro
            420                 425                 430

gcc ttc ctg ccc cag cgt cct gcc gat cag gac atc cgt gtc cct gcc 1344
Ala Phe Leu Pro Gln Arg Pro Ala Asp Gln Asp Ile Arg Val Pro Ala
        435                 440                 445

aag ggg gat ggc agc atg gag tgg ctg ggc atc aag agt ttc gac gcg 1392
Lys Gly Asp Gly Ser Met Glu Trp Leu Gly Ile Lys Ser Phe Asp Ala
    450                 455                 460

att ccc aaa gcc tac aat cca ccc cag ggc tat ctg gtc aac tgg aac 1440
Ile Pro Lys Ala Tyr Asn Pro Pro Gln Gly Tyr Leu Val Asn Trp Asn
465                 470                 475                 480

aac aag cct gcg ccg gac aaa acc aat acg gat act tac tat tgg acc 1488
Asn Lys Pro Ala Pro Asp Lys Thr Asn Thr Asp Thr Tyr Tyr Trp Thr
                485                 490                 495

tat ggc gac cgc atg aat gaa ctg gtc agt cag tac cag cag aaa gac 1536
Tyr Gly Asp Arg Met Asn Glu Leu Val Ser Gln Tyr Gln Gln Lys Asp
            500                 505                 510

ctc ttc agt gtg cag gag atc tgg gag ttc aat caa aaa gcc tcc tat 1584
Leu Phe Ser Val Gln Glu Ile Trp Glu Phe Asn Gln Lys Ala Ser Tyr
        515                 520                 525

agc gat gtg aac tgg cgc tac ttc cgc cca cat ctg gaa aag ctg gcg 1632
Ser Asp Val Asn Trp Arg Tyr Phe Arg Pro His Leu Glu Lys Leu Ala
    530                 535                 540

caa cag ctg ccg gcc gac gat agc agc aag gcg gcg ctg acg atg ttg 1680
Gln Gln Leu Pro Ala Asp Asp Ser Ser Lys Ala Ala Leu Thr Met Leu
545                 550                 555                 560

ctc gcc tgg gat gga atg gaa cag gat cag gga ggg caa aat gcc gga 1728
Leu Ala Trp Asp Gly Met Glu Gln Asp Gln Gly Gly Gln Asn Ala Gly
                565                 570                 575

ccg gcg cgg gtg ctc ttc aag acc tgg ctg gaa gaa atg tac aag cag 1776
Pro Ala Arg Val Leu Phe Lys Thr Trp Leu Glu Glu Met Tyr Lys Gln
            580                 585                 590

gtc ttg atg ccg gtg gtg cct gaa tcg cat cgc gcc atg tat agc cag 1824
Val Leu Met Pro Val Val Pro Glu Ser His Arg Ala Met Tyr Ser Gln
        595                 600                 605

act ggt ttt gcc acg cag caa ggt ccc aac ccc ggt tcc atc aac ttg 1872
Thr Gly Phe Ala Thr Gln Gln Gly Pro Asn Pro Gly Ser Ile Asn Leu
    610                 615                 620

agc atg ggc acc aag gtc ttg ttg cgt gcc ttg gtg ctg gaa gcc cat 1920
Ser Met Gly Thr Lys Val Leu Leu Arg Ala Leu Val Leu Glu Ala His
625                 630                 635                 640

ccc gat ccc aag cgt gtg aat gtc ttt ggt gag cgt tcg tct cag gaa 1968
Pro Asp Pro Lys Arg Val Asn Val Phe Gly Glu Arg Ser Ser Gln Glu
                645                 650                 655

atc atg cac aca gct ttg caa aat gcg cag gcc cgc ttg agc cag gag 2016
Ile Met His Thr Ala Leu Gln Asn Ala Gln Ala Arg Leu Ser Gln Glu
            660                 665                 670

cag ggc gct cag atg gcg cgc tgg acc atg ccg acc tcc gtg cat cgt 2064
Gln Gly Ala Gln Met Ala Arg Trp Thr Met Pro Thr Ser Val His Arg
        675                 680                 685

ttc agc gac aag aac ttc acg gga acc ccg cag acg atg cct ggc aat 2112
Phe Ser Asp Lys Asn Phe Thr Gly Thr Pro Gln Thr Met Pro Gly Asn
    690                 695                 700

acc ttt gcc ttt acc ggc tat cag aat cga ggc acg gaa aat aac cgc 2160
Thr Phe Ala Phe Thr Gly Tyr Gln Asn Arg Gly Thr Glu Asn Asn Arg
705                 710                 715                 720

gtg gtg ttt gat gcc aag ggc gtg gag ttc tgc gac gcc atg ccg ccc 2208
Val Val Phe Asp Ala Lys Gly Val Glu Phe Cys Asp Ala Met Pro Pro
                725                 730                 735

ggc caa agc ggt ttc acc gac cgc aat gga gtg cgc agc ccg cat tat 2256
Gly Gln Ser Gly Phe Thr Asp Arg Asn Gly Val Arg Ser Pro His Tyr

740                 745                 750

gag gat cag ctg aag ttg tac gag aac ttc gag tgc aag acg atg gat        2304
Glu Asp Gln Leu Lys Leu Tyr Glu Asn Phe Glu Cys Lys Thr Met Asp
        755                 760                 765

gtg acg cat gcg gac att cgt cgt aat gcg caa agc agc acg atg ctg        2352
Val Thr His Ala Asp Ile Arg Arg Asn Ala Gln Ser Ser Thr Met Leu
    770                 775                 780

ttg att cag cct cag cct taa                                            2373
Leu Ile Gln Pro Gln Pro
785                 790

<210>  2

<211>  790

<212>  PRT

<213>  Alcaligenes faecalis

<400>  2

Gln Val Gln Ser Val Glu Val Met Arg Asp Ser Tyr Gly Val Pro His
1               5                   10                  15

Val Phe Ala Asp Ser His Tyr Gly Leu Tyr Tyr Gly Tyr Gly Tyr Ala
            20                  25                  30

Val Ala Gln Asp Arg Leu Phe Gln Met Asp Met Ala Arg Arg Ser Phe
        35                  40                  45

Val Gly Thr Thr Ala Ala Val Leu Gly Pro Gly Glu Gln Asp Ala Tyr
    50                  55                  60

Val Lys Tyr Asp Met Gln Val Arg Gln Asn Phe Thr Pro Ala Ser Ile
65                  70                  75                  80

Gln Arg Gln Ile Ala Ala Leu Ser Lys Asp Glu Arg Asp Ile Phe Arg
                85                  90                  95

Gly Tyr Ala Asp Gly Tyr Asn Ala Tyr Leu Glu Gln Val Arg Arg Arg
            100                 105                 110

Pro Glu Leu Leu Pro Lys Glu Tyr Val Asp Phe Asp Phe Gln Pro Glu
        115                 120                 125

Pro Leu Thr Asp Phe Asp Val Val Met Ile Trp Val Gly Ser Met Ala
    130                 135                 140

Asn Arg Phe Ser Asp Thr Asn Leu Glu Val Thr Ala Leu Ala Met Arg
145                 150                 155                 160

Gln Ser Leu Glu Lys Gln His Gly Pro Glu Arg Gly Arg Ala Leu Phe
                165                 170                 175

Asp Glu Leu Leu Trp Ile Asn Asp Thr Thr Ala Pro Thr Thr Val Pro
            180                 185                 190

Ala Pro Ala Ala Glu His Lys Pro Gln Ala Gln Ala Gly Thr Gln Asp
        195                 200                 205

Leu Ala His Val Ser Ser Pro Val Leu Ala Thr Glu Leu Glu Arg Gln
    210                 215                 220

Asp Lys His Trp Gly Gly Arg Gly Pro Asp Phe Ala Pro Lys Ala Ser
225                 230                 235                 240

Asn Leu Trp Ser Thr Arg Pro Glu Arg Val Gln Glu Gly Ser Thr Val
                245                 250                 255

Leu Ile Asn Gly Pro Gln Phe Gly Trp Tyr Asn Pro Ala Tyr Thr Tyr
            260                 265                 270

Gly Ile Gly Leu His Gly Ala Gly Phe Asp Val Val Gly Asn Thr Pro
        275                 280                 285

Phe Ala Tyr Pro Ile Val Leu Phe Gly Thr Asn Ser Glu Ile Ala Trp
    290                 295                 300

Gly Ala Thr Ala Gly Pro Gln Asp Val Val Asp Ile Tyr Gln Glu Lys
305                 310                 315                 320

Leu Asn Pro Ser Arg Ala Asp Gln Tyr Trp Phe Asn Asn Ala Trp Arg
                325                 330                 335

Thr Met Glu Gln Arg Lys Glu Arg Ile Gln Val Arg Gly Gln Ala Asp
            340                 345                 350

Arg Glu Met Thr Ile Trp Arg Thr Val His Gly Pro Val Met Gln Phe

        355                 360                 365

Asp Tyr Asp Gln Gly Ala Ala Tyr Ser Lys Lys Arg Ser Trp Asp Gly
    370                 375                 380

Tyr Glu Val Gln Ser Leu Leu Ala Trp Leu Asn Val Ala Lys Ala Arg
385                 390                 395                 400

Asn Trp Thr Glu Phe Leu Asp Gln Ala Ser Lys Met Ala Ile Ser Ile
                405                 410                 415

Asn Trp Tyr Tyr Ala Asp Lys His Gly Asn Ile Gly Tyr Val Ser Pro
            420                 425                 430

Ala Phe Leu Pro Gln Arg Pro Ala Asp Gln Asp Ile Arg Val Pro Ala
        435                 440                 445

Lys Gly Asp Gly Ser Met Glu Trp Leu Gly Ile Lys Ser Phe Asp Ala
    450                 455                 460

Ile Pro Lys Ala Tyr Asn Pro Pro Gln Gly Tyr Leu Val Asn Trp Asn
465                 470                 475                 480

Asn Lys Pro Ala Pro Asp Lys Thr Asn Thr Asp Thr Tyr Tyr Trp Thr
                485                 490                 495

Tyr Gly Asp Arg Met Asn Glu Leu Val Ser Gln Tyr Gln Gln Lys Asp
            500                 505                 510

Leu Phe Ser Val Gln Glu Ile Trp Glu Phe Asn Gln Lys Ala Ser Tyr
        515                 520                 525

Ser Asp Val Asn Trp Arg Tyr Phe Arg Pro His Leu Glu Lys Leu Ala
    530                 535                 540

Gln Gln Leu Pro Ala Asp Asp Ser Ser Lys Ala Ala Leu Thr Met Leu
545                 550                 555                 560

Leu Ala Trp Asp Gly Met Glu Gln Asp Gln Gly Gly Gln Asn Ala Gly
                565                 570                 575

Pro Ala Arg Val Leu Phe Lys Thr Trp Leu Glu Glu Met Tyr Lys Gln
            580                 585                 590

Val Leu Met Pro Val Val Pro Glu Ser His Arg Ala Met Tyr Ser Gln
        595                 600                 605

Thr Gly Phe Ala Thr Gln Gln Gly Pro Asn Pro Gly Ser Ile Asn Leu
    610                 615                 620

Ser Met Gly Thr Lys Val Leu Leu Arg Ala Leu Val Leu Glu Ala His
625                 630                 635                 640

Pro Asp Pro Lys Arg Val Asn Val Phe Gly Glu Arg Ser Ser Gln Glu
                645                 650                 655

Ile Met His Thr Ala Leu Gln Asn Ala Gln Ala Arg Leu Ser Gln Glu
            660                 665                 670

Gln Gly Ala Gln Met Ala Arg Trp Thr Met Pro Thr Ser Val His Arg
        675                 680                 685

Phe Ser Asp Lys Asn Phe Thr Gly Thr Pro Gln Thr Met Pro Gly Asn
    690                 695                 700

Thr Phe Ala Phe Thr Gly Tyr Gln Asn Arg Gly Thr Glu Asn Asn Arg
705                 710                 715                 720

Val Val Phe Asp Ala Lys Gly Val Glu Phe Cys Asp Ala Met Pro Pro
                725                 730                 735

Gly Gln Ser Gly Phe Thr Asp Arg Asn Gly Val Arg Ser Pro His Tyr
            740                 745                 750

Glu Asp Gln Leu Lys Leu Tyr Glu Asn Phe Glu Cys Lys Thr Met Asp
        755                 760                 765

Val Thr His Ala Asp Ile Arg Arg Asn Ala Gln Ser Ser Thr Met Leu
    770                 775                 780

Leu Ile Gln Pro Gln Pro
785                 790

<210>  3

<211>  2373

<212>  DNA

<213>  Alcaligenes faecalis

<220>

<221>  CDS

<222>  (1)..(2373)

<223>  Nukleinsäure kodierend für modifizierte Pro-Penicillinamidase

<400>  3
caa gtg cag tcg gta gag gtg atg cgg gac agt tat ggc gtg ccg cac      48
Gln Val Gln Ser Val Glu Val Met Arg Asp Ser Tyr Gly Val Pro His
1               5                   10                  15

gtc ttt gcc gac agc cac tat ggc ttg tat tac ggc tat ggt tat gcg      96
Val Phe Ala Asp Ser His Tyr Gly Leu Tyr Tyr Gly Tyr Gly Tyr Ala
            20                  25                  30

gtc gcc caa gac cgt ctg ttc cag atg gac atg gcg cgt cgc tcc ttt      144
Val Ala Gln Asp Arg Leu Phe Gln Met Asp Met Ala Arg Arg Ser Phe
        35                  40                  45

gtc ggc aca acc gcc gcc gtc tta ggc cct ggt gag caa gat gcc tac      192
Val Gly Thr Thr Ala Ala Val Leu Gly Pro Gly Glu Gln Asp Ala Tyr
    50                  55                  60

gtc aag tac gac atg cag gtg cgg cag aac ttc acc ccg gct tcc ata      240
Val Lys Tyr Asp Met Gln Val Arg Gln Asn Phe Thr Pro Ala Ser Ile
65                  70                  75                  80

cag cgg cag atc gcg gcc ttg tcc aag gat gag cgc gat att ttt cgt      288
Gln Arg Gln Ile Ala Ala Leu Ser Lys Asp Glu Arg Asp Ile Phe Arg
                85                  90                  95

ggc tat gcc gat ggc tat aac gcc tat ctg gag cag gtg cgg cgt cgc      336
Gly Tyr Ala Asp Gly Tyr Asn Ala Tyr Leu Glu Gln Val Arg Arg Arg
            100                 105                 110

cct gag ttg ctg ccc aaa gaa tat gtg gat ttt gat ttc cag ccc gag      384
Pro Glu Leu Leu Pro Lys Glu Tyr Val Asp Phe Asp Phe Gln Pro Glu
        115                 120                 125

ccg ctg acc gac ttt gat gtg gtc atg atc tgg gtg ggc tcc atg gcc      432
Pro Leu Thr Asp Phe Asp Val Val Met Ile Trp Val Gly Ser Met Ala
    130                 135                 140

aat cgc ttc tcc gac acg aat ctg gaa gtg acg gca ctg gcc atg cgt      480
Asn Arg Phe Ser Asp Thr Asn Leu Glu Val Thr Ala Leu Ala Met Arg
145                 150                 155                 160

cag tct ctg gag aaa cag cac ggc ccg gaa cga ggc cgt gcc ttg ttt 528
Gln Ser Leu Glu Lys Gln His Gly Pro Glu Arg Gly Arg Ala Leu Phe
                165                 170                 175

gat gag ctg ctg tgg atc aat gac aca aca gct ccc act acg gtt ccg 576
Asp Glu Leu Leu Trp Ile Asn Asp Thr Thr Ala Pro Thr Thr Val Pro
            180                 185                 190

gcc ccc gct gcc gag cac aag ccg cag gca caa gca ggg ccg cag gat 624
Ala Pro Ala Ala Glu His Lys Pro Gln Ala Gln Ala Gly Pro Gln Asp
        195                 200                 205

ctg gct cat gtt tcc tcg cca gta ctg gct acc gag cta gag cgc cag 672
Leu Ala His Val Ser Ser Pro Val Leu Ala Thr Glu Leu Glu Arg Gln
    210                 215                 220

gac aag cac tgg ggc ggc cgt ggc ccg gac ttc gcg ccc aag gct agc 720
Asp Lys His Trp Gly Gly Arg Gly Pro Asp Phe Ala Pro Lys Ala Ser
225                 230                 235                 240

aac ctg tgg agc act cgc ccc gag cga gtg cag gag ggc tcg acc gta 768
Asn Leu Trp Ser Thr Arg Pro Glu Arg Val Gln Glu Gly Ser Thr Val
                245                 250                 255

ctg atc aac ggc cca cag ttt ggc tgg tac aac ccg gcc tac acc tat 816
Leu Ile Asn Gly Pro Gln Phe Gly Trp Tyr Asn Pro Ala Tyr Thr Tyr
            260                 265                 270

ggc att ggc ttg cat ggc gcc ggc ttc gat gtg gtg ggt aat acg cct 864
Gly Ile Gly Leu His Gly Ala Gly Phe Asp Val Val Gly Asn Thr Pro
        275                 280                 285

ttt gcc tat ccg atc gta ctg ttt ggc acc aat agc gag att gcc tgg 912
Phe Ala Tyr Pro Ile Val Leu Phe Gly Thr Asn Ser Glu Ile Ala Trp
    290                 295                 300

ggg gcg act gct ggc ccg caa gat gtg gtg gac ata tat cag gaa aaa 960
Gly Ala Thr Ala Gly Pro Gln Asp Val Val Asp Ile Tyr Gln Glu Lys
305                 310                 315                 320

ttg aac ccc tcg cgt gcc gat cag tac tgg ttc aac aat gcc tgg cgc 1008
Leu Asn Pro Ser Arg Ala Asp Gln Tyr Trp Phe Asn Asn Ala Trp Arg
                325                 330                 335

acg atg gag cag cgc aag gaa cgt atc cag gta cgc ggt cag gct gat 1056
Thr Met Glu Gln Arg Lys Glu Arg Ile Gln Val Arg Gly Gln Ala Asp
            340                 345                 350

cgg gaa atg acg atc tgg cgc acc gtg cac ggc cct gtg atg cag ttt 1104
Arg Glu Met Thr Ile Trp Arg Thr Val His Gly Pro Val Met Gln Phe
        355                 360                 365

gat tac gat cag ggc gcg gcg tac agc aag aaa cgc agc tgg gat ggc 1152
Asp Tyr Asp Gln Gly Ala Ala Tyr Ser Lys Lys Arg Ser Trp Asp Gly
    370                 375                 380

tat gag gtg cag tcc ttg cta gcc tgg ttg aac gtg gcc aag gcc cgc 1200
Tyr Glu Val Gln Ser Leu Leu Ala Trp Leu Asn Val Ala Lys Ala Arg
385                 390                 395                 400

aac tgg acg gag ttt ctg gat caa gcc agc aag atg gcg att tcg atc 1248
Asn Trp Thr Glu Phe Leu Asp Gln Ala Ser Lys Met Ala Ile Ser Ile
                405                 410                 415

aac tgg tac tac gcc gac aag cac ggc aat att ggt tat gtc tcg ccg 1296
Asn Trp Tyr Tyr Ala Asp Lys His Gly Asn Ile Gly Tyr Val Ser Pro
            420                 425                 430

gcc ttc ctg ccc cag cgt cct gcc gat cag gac atc cgt gtc cct gcc 1344
Ala Phe Leu Pro Gln Arg Pro Ala Asp Gln Asp Ile Arg Val Pro Ala
        435                 440                 445

aag ggg gat ggc agc atg gag tgg ctg ggc atc aag agt ttc gac gcg 1392
Lys Gly Asp Gly Ser Met Glu Trp Leu Gly Ile Lys Ser Phe Asp Ala
    450                 455                 460

att ccc aaa gcc tac aat cca ccc cag ggc tat ctg gtc aac tgg aac 1440
Ile Pro Lys Ala Tyr Asn Pro Pro Gln Gly Tyr Leu Val Asn Trp Asn
465                 470                 475                 480

aac aag cct gcg ccg gac aaa acc aat acg gat act tac tat tgg acc 1488
Asn Lys Pro Ala Pro Asp Lys Thr Asn Thr Asp Thr Tyr Tyr Trp Thr
                485                 490                 495

tat ggc gac cgc atg aat gaa ctg gtc agt cag tac cag cag aaa gac 1536
Tyr Gly Asp Arg Met Asn Glu Leu Val Ser Gln Tyr Gln Gln Lys Asp
            500                 505                 510

ctc ttc agt gtg cag gag atc tgg gag ttc aat caa aaa gcc tcc tat 1584
Leu Phe Ser Val Gln Glu Ile Trp Glu Phe Asn Gln Lys Ala Ser Tyr
        515                 520                 525

agc gat gtg aac tgg cgc tac ttc cgc cca cat ctg gaa aag ctg gcg 1632
Ser Asp Val Asn Trp Arg Tyr Phe Arg Pro His Leu Glu Lys Leu Ala
    530                 535                 540

caa cag ctg ccg gcc gac gat agc agc aag gcg gcg ctg acg atg ttg 1680
Gln Gln Leu Pro Ala Asp Asp Ser Ser Lys Ala Ala Leu Thr Met Leu
545                 550                 555                 560

ctc gcc tgg gat gga atg gaa cag gat cag gga ggg caa aat gcc gga 1728
Leu Ala Trp Asp Gly Met Glu Gln Asp Gln Gly Gly Gln Asn Ala Gly
                565                 570                 575

ccg gcg cgg gtg ctc ttc aag acc tgg ctg gaa gaa atg tac aag cag 1776
Pro Ala Arg Val Leu Phe Lys Thr Trp Leu Glu Glu Met Tyr Lys Gln
            580                 585                 590

gtc ttg atg ccg gtg gtg cct gaa tcg cat cgc gcc atg tat agc cag 1824
Val Leu Met Pro Val Val Pro Glu Ser His Arg Ala Met Tyr Ser Gln
        595                 600                 605

act ggt ttt gcc acg cag caa ggt ccc aac ccc ggt tcc atc aac ttg 1872
Thr Gly Phe Ala Thr Gln Gln Gly Pro Asn Pro Gly Ser Ile Asn Leu
    610                 615                 620

agc atg ggc acc aag gtc ttg ttg cgt gcc ttg gtg ctg gaa gcc cat 1920
Ser Met Gly Thr Lys Val Leu Leu Arg Ala Leu Val Leu Glu Ala His
625                 630                 635                 640

ccc gat ccc aag cgt gtg aat gtc ttt ggt gag cgt tcg tct cag gaa 1968
Pro Asp Pro Lys Arg Val Asn Val Phe Gly Glu Arg Ser Ser Gln Glu
                645                 650                 655

atc atg cac aca gct ttg caa aat gcg cag gcc cgc ttg agc cag gag 2016
Ile Met His Thr Ala Leu Gln Asn Ala Gln Ala Arg Leu Ser Gln Glu
            660                 665                 670

cag ggc gct cag atg gcg cgc tgg acc atg ccg acc tcc gtg cat cgt 2064
Gln Gly Ala Gln Met Ala Arg Trp Thr Met Pro Thr Ser Val His Arg
        675                 680                 685

ttc agc gac aag aac ttc acg gga acc ccg cag acg atg cct ggc aat 2112
Phe Ser Asp Lys Asn Phe Thr Gly Thr Pro Gln Thr Met Pro Gly Asn
    690                 695                 700

acc ttt gcc ttt acc ggc tat cag aat cga ggc acg gaa aat aac cgc 2160
Thr Phe Ala Phe Thr Gly Tyr Gln Asn Arg Gly Thr Glu Asn Asn Arg
705                 710                 715                 720

gtg gtg ttt gat gcc aag ggc gtg gag ttc tgc gac gcc atg ccg ccc 2208
Val Val Phe Asp Ala Lys Gly Val Glu Phe Cys Asp Ala Met Pro Pro
                725                 730                 735

ggc caa agc ggt ttc acc gac cgc aat gga gtg cgc agc ccg cat tat 2256
Gly Gln Ser Gly Phe Thr Asp Arg Asn Gly Val Arg Ser Pro His Tyr
            740                 745                 750

gag gat cag ctg aag ttg tac gag aac ttc gag tgc aag acg atg gat 2304
Glu Asp Gln Leu Lys Leu Tyr Glu Asn Phe Glu Cys Lys Thr Met Asp
        755                 760                 765

gtg acg cat gcg gac att cgt cgt aat gcg caa agc agc acg atg ctg 2352
Val Thr His Ala Asp Ile Arg Arg Asn Ala Gln Ser Ser Thr Met Leu
    770                 775                 780

ttg att cag cct cag cct taa 2373
Leu Ile Gln Pro Gln Pro
785                 790

<210> 4

<211> 790

<212> PRT

<213>  Alcaligenes faecalis

<400>  4

Gln Val Gln Ser Val Glu Val Met Arg Asp Ser Tyr Gly Val Pro His
1               5                   10                  15

Val Phe Ala Asp Ser His Tyr Gly Leu Tyr Tyr Gly Tyr Gly Tyr Ala
            20                  25                  30

Val Ala Gln Asp Arg Leu Phe Gln Met Asp Met Ala Arg Arg Ser Phe
        35                  40                  45

Val Gly Thr Thr Ala Ala Val Leu Gly Pro Gly Glu Gln Asp Ala Tyr
    50                  55                  60

Val Lys Tyr Asp Met Gln Val Arg Gln Asn Phe Thr Pro Ala Ser Ile
65                  70                  75                  80

Gln Arg Gln Ile Ala Ala Leu Ser Lys Asp Glu Arg Asp Ile Phe Arg
                85                  90                  95

Gly Tyr Ala Asp Gly Tyr Asn Ala Tyr Leu Glu Gln Val Arg Arg Arg
            100                 105                 110

Pro Glu Leu Leu Pro Lys Glu Tyr Val Asp Phe Asp Phe Gln Pro Glu
        115                 120                 125

Pro Leu Thr Asp Phe Asp Val Val Met Ile Trp Val Gly Ser Met Ala
    130                 135                 140

Asn Arg Phe Ser Asp Thr Asn Leu Glu Val Thr Ala Leu Ala Met Arg
145                 150                 155                 160

Gln Ser Leu Glu Lys Gln His Gly Pro Glu Arg Gly Arg Ala Leu Phe
                165                 170                 175

Asp Glu Leu Leu Trp Ile Asn Asp Thr Thr Ala Pro Thr Thr Val Pro
            180                 185                 190

Ala Pro Ala Ala Glu His Lys Pro Gln Ala Gln Ala Gly Pro Gln Asp
        195                 200                 205

Leu Ala His Val Ser Ser Pro Val Leu Ala Thr Glu Leu Glu Arg Gln
    210                 215                 220

Asp Lys His Trp Gly Gly Arg Gly Pro Asp Phe Ala Pro Lys Ala Ser
225                 230                 235                 240

Asn Leu Trp Ser Thr Arg Pro Glu Arg Val Gln Glu Gly Ser Thr Val
                245                 250                 255

Leu Ile Asn Gly Pro Gln Phe Gly Trp Tyr Asn Pro Ala Tyr Thr Tyr
            260                 265                 270

Gly Ile Gly Leu His Gly Ala Gly Phe Asp Val Val Gly Asn Thr Pro
        275                 280                 285

Phe Ala Tyr Pro Ile Val Leu Phe Gly Thr Asn Ser Glu Ile Ala Trp
    290                 295                 300

Gly Ala Thr Ala Gly Pro Gln Asp Val Val Asp Ile Tyr Gln Glu Lys
305                 310                 315                 320

Leu Asn Pro Ser Arg Ala Asp Gln Tyr Trp Phe Asn Asn Ala Trp Arg
                325                 330                 335

Thr Met Glu Gln Arg Lys Glu Arg Ile Gln Val Arg Gly Gln Ala Asp
            340                 345                 350

Arg Glu Met Thr Ile Trp Arg Thr Val His Gly Pro Val Met Gln Phe
        355                 360                 365

Asp Tyr Asp Gln Gly Ala Ala Tyr Ser Lys Lys Arg Ser Trp Asp Gly
    370                 375                 380

Tyr Glu Val Gln Ser Leu Leu Ala Trp Leu Asn Val Ala Lys Ala Arg
385                 390                 395                 400

Asn Trp Thr Glu Phe Leu Asp Gln Ala Ser Lys Met Ala Ile Ser Ile
                405                 410                 415

Asn Trp Tyr Tyr Ala Asp Lys His Gly Asn Ile Gly Tyr Val Ser Pro
            420                 425                 430

Ala Phe Leu Pro Gln Arg Pro Ala Asp Gln Asp Ile Arg Val Pro Ala

435                 440                 445

Lys Gly Asp Gly Ser Met Glu Trp Leu Gly Ile Lys Ser Phe Asp Ala
    450                 455                 460

Ile Pro Lys Ala Tyr Asn Pro Pro Gln Gly Tyr Leu Val Asn Trp Asn
465                 470                 475                 480

Asn Lys Pro Ala Pro Asp Lys Thr Asn Thr Asp Thr Tyr Tyr Trp Thr
                485                 490                 495

Tyr Gly Asp Arg Met Asn Glu Leu Val Ser Gln Tyr Gln Gln Lys Asp
            500                 505                 510

Leu Phe Ser Val Gln Glu Ile Trp Glu Phe Asn Gln Lys Ala Ser Tyr
        515                 520                 525

Ser Asp Val Asn Trp Arg Tyr Phe Arg Pro His Leu Glu Lys Leu Ala
    530                 535                 540

Gln Gln Leu Pro Ala Asp Asp Ser Ser Lys Ala Ala Leu Thr Met Leu
545                 550                 555                 560

Leu Ala Trp Asp Gly Met Glu Gln Asp Gln Gly Gly Gln Asn Ala Gly
                565                 570                 575

Pro Ala Arg Val Leu Phe Lys Thr Trp Leu Glu Glu Met Tyr Lys Gln
            580                 585                 590

Val Leu Met Pro Val Val Pro Glu Ser His Arg Ala Met Tyr Ser Gln
        595                 600                 605

Thr Gly Phe Ala Thr Gln Gln Gly Pro Asn Pro Gly Ser Ile Asn Leu
    610                 615                 620

Ser Met Gly Thr Lys Val Leu Leu Arg Ala Leu Val Leu Glu Ala His
625                 630                 635                 640

Pro Asp Pro Lys Arg Val Asn Val Phe Gly Glu Arg Ser Ser Gln Glu
                645                 650                 655

Ile Met His Thr Ala Leu Gln Asn Ala Gln Ala Arg Leu Ser Gln Glu
            660                 665                 670

Gln Gly Ala Gln Met Ala Arg Trp Thr Met Pro Thr Ser Val His Arg
        675                 680                 685

Phe Ser Asp Lys Asn Phe Thr Gly Thr Pro Gln Thr Met Pro Gly Asn
    690                 695                 700

Thr Phe Ala Phe Thr Gly Tyr Gln Asn Arg Gly Thr Glu Asn Asn Arg
705                 710                 715                 720

Val Val Phe Asp Ala Lys Gly Val Glu Phe Cys Asp Ala Met Pro Pro
                725                 730                 735

Gly Gln Ser Gly Phe Thr Asp Arg Asn Gly Val Arg Ser Pro His Tyr
            740                 745                 750

Glu Asp Gln Leu Lys Leu Tyr Glu Asn Phe Glu Cys Lys Thr Met Asp
        755                 760                 765

Val Thr His Ala Asp Ile Arg Arg Asn Ala Gln Ser Ser Thr Met Leu
    770                 775                 780

Leu Ile Gln Pro Gln Pro
785                 790

<210> 5

<211> 2373

<212> DNA

<213> Alcaligenes faecalis

<220>

<221> CDS

<222> (1)..(2373)

<223> Nukleinsäure kodierend für modifizierte Pro-Penicillinamidase

<400> 5
caa gtg cag tcg gta gag gtg atg cgg gac agt tat ggc gtg ccg cac 48
Gln Val Gln Ser Val Glu Val Met Arg Asp Ser Tyr Gly Val Pro His
1               5                   10                  15

gtc ttt gcc gac agc cac tat ggc ttg tat tac ggc tat ggt tat gcg 96
Val Phe Ala Asp Ser His Tyr Gly Leu Tyr Tyr Gly Tyr Gly Tyr Ala
            20                  25                  30

gtc gcc caa gac cgt ctg ttc cag atg gac atg gcg cgt cgc tcc ttt 144
Val Ala Gln Asp Arg Leu Phe Gln Met Asp Met Ala Arg Arg Ser Phe
        35                  40                  45

gtc ggc aca acc gcc gcc gtc tta ggc cct ggt gag caa gat gcc tac 192
Val Gly Thr Thr Ala Ala Val Leu Gly Pro Gly Glu Gln Asp Ala Tyr
    50                  55                  60

gtc aag tac gac atg cag gtg cgg cag aac ttc acc ccg gct tcc ata 240
Val Lys Tyr Asp Met Gln Val Arg Gln Asn Phe Thr Pro Ala Ser Ile
65                  70                  75                  80

cag cgg cag atc gcg gcc ttg tcc aag gat gag cgc gat att ttt cgt 288
Gln Arg Gln Ile Ala Ala Leu Ser Lys Asp Glu Arg Asp Ile Phe Arg
                85                  90                  95

ggc tat gcc gat ggc tat aac gcc tat ctg gag cag gtg cgg cgt cgc 336
Gly Tyr Ala Asp Gly Tyr Asn Ala Tyr Leu Glu Gln Val Arg Arg Arg
            100                 105                 110

cct gag ttg ctg ccc aaa gaa tat gtg gat ttt gat ttc cag ccc gag 384
Pro Glu Leu Leu Pro Lys Glu Tyr Val Asp Phe Asp Phe Gln Pro Glu
        115                 120                 125

ccg ctg acc gac ttt gat gtg gtc atg atc tgg gtg ggc tcc atg gcc 432
Pro Leu Thr Asp Phe Asp Val Val Met Ile Trp Val Gly Ser Met Ala
    130                 135                 140

aat cgc ttc tcc gac acg aat ctg gaa gtg acg gca ctg gcc atg cgt 480
Asn Arg Phe Ser Asp Thr Asn Leu Glu Val Thr Ala Leu Ala Met Arg
145                 150                 155                 160

cag tct ctg gag aaa cag cac ggc ccg gaa cga ggc cgt gcc ttg ttt 528
Gln Ser Leu Glu Lys Gln His Gly Pro Glu Arg Gly Arg Ala Leu Phe
                165                 170                 175

gat gag ctg ctg tgg atc aat gac aca aca gct ccc act acg gtt ccg 576
Asp Glu Leu Leu Trp Ile Asn Asp Thr Thr Ala Pro Thr Thr Val Pro
            180                 185                 190

gcc ccc gct gcc gag cac aag ccg cag gca caa gca ggg ggc cag gat 624
Ala Pro Ala Ala Glu His Lys Pro Gln Ala Gln Ala Gly Gly Gln Asp
        195                 200                 205

ctg gct cat gtt tcc tcg cca gta ctg gct acc gag cta gag cgc cag 672
Leu Ala His Val Ser Ser Pro Val Leu Ala Thr Glu Leu Glu Arg Gln
    210                 215                 220

gac aag cac tgg ggc ggc cgt ggc ccg gac ttc gcg ccc aag gct agc 720
Asp Lys His Trp Gly Gly Arg Gly Pro Asp Phe Ala Pro Lys Ala Ser
225                 230                 235                 240

aac ctg tgg agc act cgc ccc gag cga gtg cag gag ggc tcg acc gta 768

Asn Leu Trp Ser Thr Arg Pro Glu Arg Val Gln Glu Gly Ser Thr Val
                245                 250                 255

ctg atc aac ggc cca cag ttt ggc tgg tac aac ccg gcc tac acc tat 816
Leu Ile Asn Gly Pro Gln Phe Gly Trp Tyr Asn Pro Ala Tyr Thr Tyr
            260                 265                 270

ggc att ggc ttg cat ggc gcc ggc ttc gat gtg gtg ggt aat acg cct 864
Gly Ile Gly Leu His Gly Ala Gly Phe Asp Val Val Gly Asn Thr Pro
        275                 280                 285

ttt gcc tat ccg atc gta ctg ttt ggc acc aat agc gag att gcc tgg 912
Phe Ala Tyr Pro Ile Val Leu Phe Gly Thr Asn Ser Glu Ile Ala Trp
    290                 295                 300

ggg gcg act gct ggc ccg caa gat gtg gtg gac ata tat cag gaa aaa 960
Gly Ala Thr Ala Gly Pro Gln Asp Val Val Asp Ile Tyr Gln Glu Lys
305                 310                 315                 320

ttg aac ccc tcg cgt gcc gat cag tac tgg ttc aac aat gcc tgg cgc 1008
Leu Asn Pro Ser Arg Ala Asp Gln Tyr Trp Phe Asn Asn Ala Trp Arg
                325                 330                 335

acg atg gag cag cgc aag gaa cgt atc cag gta cgc ggt cag gct gat 1056
Thr Met Glu Gln Arg Lys Glu Arg Ile Gln Val Arg Gly Gln Ala Asp
            340                 345                 350

cgg gaa atg acg atc tgg cgc acc gtg cac ggc cct gtg atg cag ttt 1104
Arg Glu Met Thr Ile Trp Arg Thr Val His Gly Pro Val Met Gln Phe
        355                 360                 365

gat tac gat cag ggc gcg gcg tac agc aag aaa cgc agc tgg gat ggc 1152
Asp Tyr Asp Gln Gly Ala Ala Tyr Ser Lys Lys Arg Ser Trp Asp Gly
    370                 375                 380

tat gag gtg cag tcc ttg cta gcc tgg ttg aac gtg gcc aag gcc cgc 1200
Tyr Glu Val Gln Ser Leu Leu Ala Trp Leu Asn Val Ala Lys Ala Arg
385                 390                 395                 400

aac tgg acg gag ttt ctg gat caa gcc agc aag atg gcg att tcg atc 1248
Asn Trp Thr Glu Phe Leu Asp Gln Ala Ser Lys Met Ala Ile Ser Ile
                405                 410                 415

aac tgg tac tac gcc gac aag cac ggc aat att ggt tat gtc tcg ccg 1296
Asn Trp Tyr Tyr Ala Asp Lys His Gly Asn Ile Gly Tyr Val Ser Pro
            420                 425                 430

gcc ttc ctg ccc cag cgt cct gcc gat cag gac atc cgt gtc cct gcc 1344
Ala Phe Leu Pro Gln Arg Pro Ala Asp Gln Asp Ile Arg Val Pro Ala
        435                 440                 445

aag ggg gat ggc agc atg gag tgg ctg ggc atc aag agt ttc gac gcg 1392
Lys Gly Asp Gly Ser Met Glu Trp Leu Gly Ile Lys Ser Phe Asp Ala
    450                 455                 460

att ccc aaa gcc tac aat cca ccc cag ggc tat ctg gtc aac tgg aac 1440
Ile Pro Lys Ala Tyr Asn Pro Pro Gln Gly Tyr Leu Val Asn Trp Asn
465                 470                 475                 480

aac aag cct gcg ccg gac aaa acc aat acg gat act tac tat tgg acc 1488
Asn Lys Pro Ala Pro Asp Lys Thr Asn Thr Asp Thr Tyr Tyr Trp Thr
                485                 490                 495

tat ggc gac cgc atg aat gaa ctg gtc agt cag tac cag cag aaa gac 1536
Tyr Gly Asp Arg Met Asn Glu Leu Val Ser Gln Tyr Gln Gln Lys Asp
            500                 505                 510

ctc ttc agt gtg cag gag atc tgg gag ttc aat caa aaa gcc tcc tat 1584
Leu Phe Ser Val Gln Glu Ile Trp Glu Phe Asn Gln Lys Ala Ser Tyr
        515                 520                 525

agc gat gtg aac tgg cgc tac ttc cgc cca cat ctg gaa aag ctg gcg 1632
Ser Asp Val Asn Trp Arg Tyr Phe Arg Pro His Leu Glu Lys Leu Ala
    530                 535                 540

caa cag ctg ccg gcc gac gat agc agc aag gcg gcg ctg acg atg ttg 1680
Gln Gln Leu Pro Ala Asp Asp Ser Ser Lys Ala Ala Leu Thr Met Leu
545                 550                 555                 560

ctc gcc tgg gat gga atg gaa cag gat cag gga ggg caa aat gcc gga 1728
Leu Ala Trp Asp Gly Met Glu Gln Asp Gln Gly Gly Gln Asn Ala Gly
                565                 570                 575

ccg gcg cgg gtg ctc ttc aag acc tgg ctg gaa gaa atg tac aag cag 1776
Pro Ala Arg Val Leu Phe Lys Thr Trp Leu Glu Glu Met Tyr Lys Gln
            580                 585                 590

gtc ttg atg ccg gtg gtg cct gaa tcg cat cgc gcc atg tat agc cag 1824
Val Leu Met Pro Val Val Pro Glu Ser His Arg Ala Met Tyr Ser Gln
        595                 600                 605

act ggt ttt gcc acg cag caa ggt ccc aac ccc ggt tcc atc aac ttg 1872
Thr Gly Phe Ala Thr Gln Gln Gly Pro Asn Pro Gly Ser Ile Asn Leu
    610                 615                 620

agc atg ggc acc aag gtc ttg ttg cgt gcc ttg gtg ctg gaa gcc cat 1920
Ser Met Gly Thr Lys Val Leu Leu Arg Ala Leu Val Leu Glu Ala His
625                 630                 635                 640

ccc gat ccc aag cgt gtg aat gtc ttt ggt gag cgt tcg tct cag gaa 1968
Pro Asp Pro Lys Arg Val Asn Val Phe Gly Glu Arg Ser Ser Gln Glu
                645                 650                 655

atc atg cac aca gct ttg caa aat gcg cag gcc cgc ttg agc cag gag 2016
Ile Met His Thr Ala Leu Gln Asn Ala Gln Ala Arg Leu Ser Gln Glu
            660                 665                 670

cag ggc gct cag atg gcg cgc tgg acc atg ccg acc tcc gtg cat cgt 2064
Gln Gly Ala Gln Met Ala Arg Trp Thr Met Pro Thr Ser Val His Arg
        675                 680                 685

ttc agc gac aag aac ttc acg gga acc ccg cag acg atg cct ggc aat 2112
Phe Ser Asp Lys Asn Phe Thr Gly Thr Pro Gln Thr Met Pro Gly Asn
    690                 695                 700

acc ttt gcc ttt acc ggc tat cag aat cga ggc acg gaa aat aac cgc 2160

Thr Phe Ala Phe Thr Gly Tyr Gln Asn Arg Gly Thr Glu Asn Asn Arg
705                 710                 715                 720

gtg gtg ttt gat gcc aag ggc gtg gag ttc tgc gac gcc atg ccg ccc      2208
Val Val Phe Asp Ala Lys Gly Val Glu Phe Cys Asp Ala Met Pro Pro
                725                 730                 735

ggc caa agc ggt ttc acc gac cgc aat gga gtg cgc agc ccg cat tat      2256
Gly Gln Ser Gly Phe Thr Asp Arg Asn Gly Val Arg Ser Pro His Tyr
            740                 745                 750

gag gat cag ctg aag ttg tac gag aac ttc gag tgc aag acg atg gat      2304
Glu Asp Gln Leu Lys Leu Tyr Glu Asn Phe Glu Cys Lys Thr Met Asp
        755                 760                 765

gtg acg cat gcg gac att cgt cgt aat gcg caa agc agc acg atg ctg      2352
Val Thr His Ala Asp Ile Arg Arg Asn Ala Gln Ser Ser Thr Met Leu
    770                 775                 780

ttg att cag cct cag cct taa                                          2373
Leu Ile Gln Pro Gln Pro
785                 790

<210>  6

<211>  790

<212>  PRT

<213>  Alcaligenes faecalis

<400>  6

Gln Val Gln Ser Val Glu Val Met Arg Asp Ser Tyr Gly Val Pro His
1               5                   10                  15

Val Phe Ala Asp Ser His Tyr Gly Leu Tyr Tyr Gly Tyr Gly Tyr Ala
            20                  25                  30

Val Ala Gln Asp Arg Leu Phe Gln Met Asp Met Ala Arg Arg Ser Phe
        35                  40                  45

Val Gly Thr Thr Ala Ala Val Leu Gly Pro Gly Glu Gln Asp Ala Tyr
    50                  55                  60

Val Lys Tyr Asp Met Gln Val Arg Gln Asn Phe Thr Pro Ala Ser Ile
65                  70                  75                  80

Gln Arg Gln Ile Ala Ala Leu Ser Lys Asp Glu Arg Asp Ile Phe Arg
                85                  90                  95

Gly Tyr Ala Asp Gly Tyr Asn Ala Tyr Leu Glu Gln Val Arg Arg Arg
            100                 105                 110

Pro Glu Leu Leu Pro Lys Glu Tyr Val Asp Phe Asp Phe Gln Pro Glu
        115                 120                 125

Pro Leu Thr Asp Phe Asp Val Val Met Ile Trp Val Gly Ser Met Ala
    130                 135                 140

Asn Arg Phe Ser Asp Thr Asn Leu Glu Val Thr Ala Leu Ala Met Arg
145                 150                 155                 160

Gln Ser Leu Glu Lys Gln His Gly Pro Glu Arg Gly Arg Ala Leu Phe
                165                 170                 175

Asp Glu Leu Leu Trp Ile Asn Asp Thr Thr Ala Pro Thr Thr Val Pro
            180                 185                 190

Ala Pro Ala Ala Glu His Lys Pro Gln Ala Gln Ala Gly Gly Gln Asp
        195                 200                 205

Leu Ala His Val Ser Ser Pro Val Leu Ala Thr Glu Leu Glu Arg Gln
    210                 215                 220

Asp Lys His Trp Gly Gly Arg Gly Pro Asp Phe Ala Pro Lys Ala Ser
225                 230                 235                 240

Asn Leu Trp Ser Thr Arg Pro Glu Arg Val Gln Glu Gly Ser Thr Val
                245                 250                 255

Leu Ile Asn Gly Pro Gln Phe Gly Trp Tyr Asn Pro Ala Tyr Thr Tyr
            260                 265                 270

Gly Ile Gly Leu His Gly Ala Gly Phe Asp Val Val Gly Asn Thr Pro
        275                 280                 285

Phe Ala Tyr Pro Ile Val Leu Phe Gly Thr Asn Ser Glu Ile Ala Trp
    290                 295                 300

Gly Ala Thr Ala Gly Pro Gln Asp Val Val Asp Ile Tyr Gln Glu Lys
305                 310                 315                 320

Leu Asn Pro Ser Arg Ala Asp Gln Tyr Trp Phe Asn Asn Ala Trp Arg
                325                 330                 335

Thr Met Glu Gln Arg Lys Glu Arg Ile Gln Val Arg Gly Gln Ala Asp
            340                 345                 350

Arg Glu Met Thr Ile Trp Arg Thr Val His Gly Pro Val Met Gln Phe
        355                 360                 365

Asp Tyr Asp Gln Gly Ala Ala Tyr Ser Lys Lys Arg Ser Trp Asp Gly
    370                 375                 380

Tyr Glu Val Gln Ser Leu Leu Ala Trp Leu Asn Val Ala Lys Ala Arg
385                 390                 395                 400

Asn Trp Thr Glu Phe Leu Asp Gln Ala Ser Lys Met Ala Ile Ser Ile
                405                 410                 415

Asn Trp Tyr Tyr Ala Asp Lys His Gly Asn Ile Gly Tyr Val Ser Pro
            420                 425                 430

Ala Phe Leu Pro Gln Arg Pro Ala Asp Gln Asp Ile Arg Val Pro Ala
        435                 440                 445

Lys Gly Asp Gly Ser Met Glu Trp Leu Gly Ile Lys Ser Phe Asp Ala
    450                 455                 460

Ile Pro Lys Ala Tyr Asn Pro Pro Gln Gly Tyr Leu Val Asn Trp Asn
465                 470                 475                 480

Asn Lys Pro Ala Pro Asp Lys Thr Asn Thr Asp Thr Tyr Tyr Trp Thr

                485                 490                 495

Tyr Gly Asp Arg Met Asn Glu Leu Val Ser Gln Tyr Gln Gln Lys Asp
            500                 505                 510

Leu Phe Ser Val Gln Glu Ile Trp Glu Phe Asn Gln Lys Ala Ser Tyr
        515                 520                 525

Ser Asp Val Asn Trp Arg Tyr Phe Arg Pro His Leu Glu Lys Leu Ala
    530                 535                 540

Gln Gln Leu Pro Ala Asp Asp Ser Ser Lys Ala Ala Leu Thr Met Leu

545                 550                 555                 560

Leu Ala Trp Asp Gly Met Glu Gln Asp Gln Gly Gly Gln Asn Ala Gly
                565                 570                 575

Pro Ala Arg Val Leu Phe Lys Thr Trp Leu Glu Glu Met Tyr Lys Gln
            580                 585                 590

Val Leu Met Pro Val Val Pro Glu Ser His Arg Ala Met Tyr Ser Gln
        595                 600                 605

Thr Gly Phe Ala Thr Gln Gln Gly Pro Asn Pro Gly Ser Ile Asn Leu
    610                 615                 620

Ser Met Gly Thr Lys Val Leu Leu Arg Ala Leu Val Leu Glu Ala His
625                 630                 635                 640

Pro Asp Pro Lys Arg Val Asn Val Phe Gly Glu Arg Ser Ser Gln Glu
                645                 650                 655

Ile Met His Thr Ala Leu Gln Asn Ala Gln Ala Arg Leu Ser Gln Glu
            660                 665                 670

Gln Gly Ala Gln Met Ala Arg Trp Thr Met Pro Thr Ser Val His Arg
        675                 680                 685

Phe Ser Asp Lys Asn Phe Thr Gly Thr Pro Gln Thr Met Pro Gly Asn
    690                 695                 700

Thr Phe Ala Phe Thr Gly Tyr Gln Asn Arg Gly Thr Glu Asn Asn Arg
705                 710                 715                 720

Val Val Phe Asp Ala Lys Gly Val Glu Phe Cys Asp Ala Met Pro Pro
                725                 730                 735

Gly Gln Ser Gly Phe Thr Asp Arg Asn Gly Val Arg Ser Pro His Tyr
            740                 745                 750

Glu Asp Gln Leu Lys Leu Tyr Glu Asn Phe Glu Cys Lys Thr Met Asp
        755                 760                 765

Val Thr His Ala Asp Ile Arg Arg Asn Ala Gln Ser Ser Thr Met Leu
    770                 775                 780

Leu Ile Gln Pro Gln Pro
785                 790

<210>  7

<211>  2373

<212>  DNA

<213>  Alcaligenes faecalis

<220>

<221>  CDS

<222>  (1)..(2373)

<223>  Nukleinsäure kodierend für modifizierte Pro-Penicillinamidase

<400>  7
caa gtg cag tcg gta gag gtg atg cgg gac agt tat ggc gtg ccg cac      48
Gln Val Gln Ser Val Glu Val Met Arg Asp Ser Tyr Gly Val Pro His
1               5                   10                  15

gtc ttt gcc gac agc cac tat ggc ttg tat tac ggc tat ggt tat gcg      96
Val Phe Ala Asp Ser His Tyr Gly Leu Tyr Tyr Gly Tyr Gly Tyr Ala
            20                  25                  30

gtc gcc caa gac cgt ctg ttc cag atg gac atg gcg cgt cgc tcc ttt      144
Val Ala Gln Asp Arg Leu Phe Gln Met Asp Met Ala Arg Arg Ser Phe
        35                  40                  45

gtc ggc aca acc gcc gcc gtc tta ggc cct ggt gag caa gat gcc tac      192
Val Gly Thr Thr Ala Ala Val Leu Gly Pro Gly Glu Gln Asp Ala Tyr
    50                  55                  60

gtc aag tac gac atg cag gtg cgg cag aac ttc acc ccg gct tcc ata      240
Val Lys Tyr Asp Met Gln Val Arg Gln Asn Phe Thr Pro Ala Ser Ile
65                  70                  75                  80

cag cgg cag atc gcg gcc ttg tcc aag gat gag cgc gat att ttt cgt      288
Gln Arg Gln Ile Ala Ala Leu Ser Lys Asp Glu Arg Asp Ile Phe Arg
                85                  90                  95

ggc tat gcc gat ggc tat aac gcc tat ctg gag cag gtg cgg cgt cgc      336
Gly Tyr Ala Asp Gly Tyr Asn Ala Tyr Leu Glu Gln Val Arg Arg Arg
            100                 105                 110

cct gag ttg ctg ccc aaa gaa tat gtg gat ttt gat ttc cag ccc gag      384
Pro Glu Leu Leu Pro Lys Glu Tyr Val Asp Phe Asp Phe Gln Pro Glu
        115                 120                 125

ccg ctg acc gac ttt gat gtg gtc atg atc tgg gtg ggc tcc atg gcc 432
Pro Leu Thr Asp Phe Asp Val Val Met Ile Trp Val Gly Ser Met Ala
    130                 135                 140

aat cgc ttc tcc gac acg aat ctg gaa gtg acg gca ctg gcc atg cgt 480
Asn Arg Phe Ser Asp Thr Asn Leu Glu Val Thr Ala Leu Ala Met Arg
145                 150                 155                 160

cag tct ctg gag aaa cag cac ggc ccg gaa cga ggc cgt gcc ttg ttt 528
Gln Ser Leu Glu Lys Gln His Gly Pro Glu Arg Gly Arg Ala Leu Phe
                165                 170                 175

gat gag ctg ctg tgg atc aat gac aca aca gct ccc act acg gtt ccg 576
Asp Glu Leu Leu Trp Ile Asn Asp Thr Thr Ala Pro Thr Thr Val Pro
            180                 185                 190

gcc ccc gct gcc gag cac aag ccg cag gca caa gca ggg ggc cag gat 624
Ala Pro Ala Ala Glu His Lys Pro Gln Ala Gln Ala Gly Gly Gln Asp
        195                 200                 205

ctg gct cat gtt ggc tcg cca gtg ctg gct acc gag cta gag cgc cag 672
Leu Ala His Val Gly Ser Pro Val Leu Ala Thr Glu Leu Glu Arg Gln
    210                 215                 220

gac aag cac tgg ggc ggc cgt ggc ccg gac ttc gcg ccc aag gct agc 720
Asp Lys His Trp Gly Gly Arg Gly Pro Asp Phe Ala Pro Lys Ala Ser
225                 230                 235                 240

aac ctg tgg agc act cgc ccc gag cga gtg cag gag ggc tcg acc gta 768
Asn Leu Trp Ser Thr Arg Pro Glu Arg Val Gln Glu Gly Ser Thr Val
                245                 250                 255

ctg atc aac ggc cca cag ttt ggc tgg tac aac ccg gcc tac acc tat 816
Leu Ile Asn Gly Pro Gln Phe Gly Trp Tyr Asn Pro Ala Tyr Thr Tyr
            260                 265                 270

ggc att ggc ttg cat ggc gcc ggc ttc gat gtg gtg ggt aat acg cct 864
Gly Ile Gly Leu His Gly Ala Gly Phe Asp Val Val Gly Asn Thr Pro
        275                 280                 285

ttt gcc tat ccg atc gta ctg ttt ggc acc aat agc gag att gcc tgg 912
Phe Ala Tyr Pro Ile Val Leu Phe Gly Thr Asn Ser Glu Ile Ala Trp
    290                 295                 300

ggg gcg act gct ggc ccg caa gat gtg gtg gac ata tat cag gaa aaa 960
Gly Ala Thr Ala Gly Pro Gln Asp Val Val Asp Ile Tyr Gln Glu Lys
305                 310                 315                 320

ttg aac ccc tcg cgt gcc gat cag tac tgg ttc aac aat gcc tgg cgc 1008
Leu Asn Pro Ser Arg Ala Asp Gln Tyr Trp Phe Asn Asn Ala Trp Arg
                325                 330                 335

acg atg gag cag cgc aag gaa cgt atc cag gta cgc ggt cag gct gat 1056
Thr Met Glu Gln Arg Lys Glu Arg Ile Gln Val Arg Gly Gln Ala Asp
            340                 345                 350

cgg gaa atg acg atc tgg cgc acc gtg cac ggc cct gtg atg cag ttt 1104
Arg Glu Met Thr Ile Trp Arg Thr Val His Gly Pro Val Met Gln Phe

355                 360                 365

gat tac gat cag ggc gcg gcg tac agc aag aaa cgc agc tgg gat ggc      1152
Asp Tyr Asp Gln Gly Ala Ala Tyr Ser Lys Lys Arg Ser Trp Asp Gly
    370                 375                 380

tat gag gtg cag tcc ttg cta gcc tgg ttg aac gtg gcc aag gcc cgc      1200
Tyr Glu Val Gln Ser Leu Leu Ala Trp Leu Asn Val Ala Lys Ala Arg
385                 390                 395                 400

aac tgg acg gag ttt ctg gat caa gcc agc aag atg gcg att tcg atc      1248
Asn Trp Thr Glu Phe Leu Asp Gln Ala Ser Lys Met Ala Ile Ser Ile
                405                 410                 415

aac tgg tac tac gcc gac aag cac ggc aat att ggt tat gtc tcg ccg      1296
Asn Trp Tyr Tyr Ala Asp Lys His Gly Asn Ile Gly Tyr Val Ser Pro
            420                 425                 430

gcc ttc ctg ccc cag cgt cct gcc gat cag gac atc cgt gtc cct gcc      1344
Ala Phe Leu Pro Gln Arg Pro Ala Asp Gln Asp Ile Arg Val Pro Ala
        435                 440                 445

aag ggg gat ggc agc atg gag tgg ctg ggc atc aag agt ttc gac gcg      1392
Lys Gly Asp Gly Ser Met Glu Trp Leu Gly Ile Lys Ser Phe Asp Ala
    450                 455                 460

att ccc aaa gcc tac aat cca ccc cag ggc tat ctg gtc aac tgg aac      1440
Ile Pro Lys Ala Tyr Asn Pro Pro Gln Gly Tyr Leu Val Asn Trp Asn
465                 470                 475                 480

aac aag cct gcg ccg gac aaa acc aat acg gat act tac tat tgg acc      1488
Asn Lys Pro Ala Pro Asp Lys Thr Asn Thr Asp Thr Tyr Tyr Trp Thr
                485                 490                 495

tat ggc gac cgc atg aat gaa ctg gtc agt cag tac cag cag aaa gac      1536
Tyr Gly Asp Arg Met Asn Glu Leu Val Ser Gln Tyr Gln Gln Lys Asp
            500                 505                 510

ctc ttc agt gtg cag gag atc tgg gag ttc aat caa aaa gcc tcc tat      1584
Leu Phe Ser Val Gln Glu Ile Trp Glu Phe Asn Gln Lys Ala Ser Tyr
        515                 520                 525

agc gat gtg aac tgg cgc tac ttc cgc cca cat ctg gaa aag ctg gcg      1632
Ser Asp Val Asn Trp Arg Tyr Phe Arg Pro His Leu Glu Lys Leu Ala
    530                 535                 540

caa cag ctg ccg gcc gac gat agc agc aag gcg gcg ctg acg atg ttg      1680
Gln Gln Leu Pro Ala Asp Asp Ser Ser Lys Ala Aia Leu Thr Met Leu
545                 550                 555                 560

ctc gcc tgg gat gga atg gaa cag gat cag gga ggg caa aat gcc gga      1728
Leu Ala Trp Asp Gly Met Glu Gln Asp Gln Gly Gly Gln Asn Ala Gly
                565                 570                 575

ccg gcg cgg gtg ctc ttc aag acc tgg ctg gaa gaa atg tac aag cag      1776
Pro Ala Arg Val Leu Phe Lys Thr Trp Leu Glu Glu Met Tyr Lys Gln
            580                 585                 590

gtc ttg atg ccg gtg gtg cct gaa tcg cat cgc gcc atg tat agc cag     1824
Val Leu Met Pro Val Val Pro Glu Ser His Arg Ala Met Tyr Ser Gln
        595                 600                 605

act ggt ttt gcc acg cag caa ggt ccc aac ccc ggt tcc atc aac ttg     1872
Thr Gly Phe Ala Thr Gln Gln Gly Pro Asn Pro Gly Ser Ile Asn Leu
    610                 615                 620

agc atg ggc acc aag gtc ttg ttg cgt gcc ttg gtg ctg gaa gcc cat     1920
Ser Met Gly Thr Lys Val Leu Leu Arg Ala Leu Val Leu Glu Ala His
625                 630                 635                 640

ccc gat ccc aag cgt gtg aat gtc ttt ggt gag cgt tcg tct cag gaa     1968
Pro Asp Pro Lys Arg Val Asn Val Phe Gly Glu Arg Ser Ser Gln Glu
                645                 650                 655

atc atg cac aca gct ttg caa aat gcg cag gcc cgc ttg agc cag gag     2016
Ile Met His Thr Ala Leu Gln Asn Ala Gln Ala Arg Leu Ser Gln Glu
            660                 665                 670

cag ggc gct cag atg gcg cgc tgg acc atg ccg acc tcc gtg cat cgt     2064
Gln Gly Ala Gln Met Ala Arg Trp Thr Met Pro Thr Ser Val His Arg
        675                 680                 685

ttc agc gac aag aac ttc acg gga acc ccg cag acg atg cct ggc aat     2112
Phe Ser Asp Lys Asn Phe Thr Gly Thr Pro Gln Thr Met Pro Gly Asn
    690                 695                 700

acc ttt gcc ttt acc ggc tat cag aat cga ggc acg gaa aat aac cgc     2160
Thr Phe Ala Phe Thr Gly Tyr Gln Asn Arg Gly Thr Glu Asn Asn Arg
705                 710                 715                 720

gtg gtg ttt gat gcc aag ggc gtg gag ttc tgc gac gcc atg ccg ccc     2208
Val Val Phe Asp Ala Lys Gly Val Glu Phe Cys Asp Ala Met Pro Pro
                725                 730                 735

ggc caa agc ggt ttc acc gac cgc aat gga gtg cgc agc ccg cat tat     2256
Gly Gln Ser Gly Phe Thr Asp Arg Asn Gly Val Arg Ser Pro His Tyr
            740                 745                 750

gag gat cag ctg aag ttg tac gag aac ttc gag tgc aag acg atg gat     2304
Glu Asp Gln Leu Lys Leu Tyr Glu Asn Phe Glu Cys Lys Thr Met Asp
        755                 760                 765

gtg acg cat gcg gac att cgt cgt aat gcg caa agc agc acg atg ctg     2352
Val Thr His Ala Asp Ile Arg Arg Asn Ala Gln Ser Ser Thr Met Leu
    770                 775                 780

ttg att cag cct cag cct taa                                         2373
Leu Ile Gln Pro Gln Pro
785                 790

<210>  8

<211>  790

<212>  PRT

<213>  Alcaligenes faecalis

<400>  8

Gln Val Gln Ser Val Glu Val Met Arg Asp Ser Tyr Gly Val Pro His
1               5                   10                  15

Val Phe Ala Asp Ser His Tyr Gly Leu Tyr Tyr Gly Tyr Gly Tyr Ala
            20                  25                  30

Val Ala Gln Asp Arg Leu Phe Gln Met Asp Met Ala Arg Arg Ser Phe
        35                  40                  45

Val Gly Thr Thr Ala Ala Val Leu Gly Pro Gly Glu Gln Asp Ala Tyr
    50                  55                  60

Val Lys Tyr Asp Met Gln Val Arg Gln Asn Phe Thr Pro Ala Ser Ile
65                  70                  75                  80

Gln Arg Gln Ile Ala Ala Leu Ser Lys Asp Glu Arg Asp Ile Phe Arg
                85                  90                  95

Gly Tyr Ala Asp Gly Tyr Asn Ala Tyr Leu Glu Gln Val Arg Arg Arg
            100                 105                 110

Pro Glu Leu Leu Pro Lys Glu Tyr Val Asp Phe Asp Phe Gln Pro Glu
        115                 120                 125

Pro Leu Thr Asp Phe Asp Val Val Met Ile Trp Val Gly Ser Met Ala
    130                 135                 140

Asn Arg Phe Ser Asp Thr Asn Leu Glu Val Thr Ala Leu Ala Met Arg
145                 150                 155                 160

Gln Ser Leu Glu Lys Gln His Gly Pro Glu Arg Gly Arg Ala Leu Phe
                165                 170                 175

Asp Glu Leu Leu Trp Ile Asn Asp Thr Thr Ala Pro Thr Thr Val Pro
            180                 185                 190

Ala Pro Ala Ala Glu His Lys Pro Gln Ala Gln Ala Gly Gly Gln Asp
        195                 200                 205

Leu Ala His Val Gly Ser Pro Val Leu Ala Thr Glu Leu Glu Arg Gln
    210                 215                 220

Asp Lys His Trp Gly Gly Arg Gly Pro Asp Phe Ala Pro Lys Ala Ser
225                 230                 235                 240

Asn Leu Trp Ser Thr Arg Pro Glu Arg Val Gln Glu Gly Ser Thr Val
                245                 250                 255

Leu Ile Asn Gly Pro Gln Phe Gly Trp Tyr Asn Pro Ala Tyr Thr Tyr
            260                 265                 270

Gly Ile Gly Leu His Gly Ala Gly Phe Asp Val Val Gly Asn Thr Pro
        275                 280                 285

Phe Ala Tyr Pro Ile Val Leu Phe Gly Thr Asn Ser Glu Ile Ala Trp
    290                 295                 300

Gly Ala Thr Ala Gly Pro Gln Asp Val Val Asp Ile Tyr Gln Glu Lys
305                 310                 315                 320

Leu Asn Pro Ser Arg Ala Asp Gln Tyr Trp Phe Asn Asn Ala Trp Arg
                325                 330                 335

Thr Met Glu Gln Arg Lys Glu Arg Ile Gln Val Arg Gly Gln Ala Asp
            340                 345                 350

Arg Glu Met Thr Ile Trp Arg Thr Val His Gly Pro Val Met Gln Phe
        355                 360                 365

Asp Tyr Asp Gln Gly Ala Ala Tyr Ser Lys Lys Arg Ser Trp Asp Gly
    370                 375                 380

Tyr Glu Val Gln Ser Leu Leu Ala Trp Leu Asn Val Ala Lys Ala Arg
385                 390                 395                 400

Asn Trp Thr Glu Phe Leu Asp Gln Ala Ser Lys Met Ala Ile Ser Ile
                405                 410                 415

Asn Trp Tyr Tyr Ala Asp Lys His Gly Asn Ile Gly Tyr Val Ser Pro
            420                 425                 430

Ala Phe Leu Pro Gln Arg Pro Ala Asp Gln Asp Ile Arg Val Pro Ala
        435                 440                 445

Lys Gly Asp Gly Ser Met Glu Trp Leu Gly Ile Lys Ser Phe Asp Ala
    450                 455                 460

Ile Pro Lys Ala Tyr Asn Pro Pro Gln Gly Tyr Leu Val Asn Trp Asn
465                 470                 475                 480

Asn Lys Pro Ala Pro Asp Lys Thr Asn Thr Asp Thr Tyr Tyr Trp Thr
                485                 490                 495

Tyr Gly Asp Arg Met Asn Glu Leu Val Ser Gln Tyr Gln Gln Lys Asp
            500                 505                 510

Leu Phe Ser Val Gln Glu Ile Trp Glu Phe Asn Gln Lys Ala Ser Tyr
        515                 520                 525

Ser Asp Val Asn Trp Arg Tyr Phe Arg Pro His Leu Glu Lys Leu Ala
    530                 535                 540

Gln Gln Leu Pro Ala Asp Asp Ser Ser Lys Ala Ala Leu Thr Met Leu
545                 550                 555                 560

Leu Ala Trp Asp Gly Met Glu Gln Asp Gln Gly Gly Gln Asn Ala Gly
                565                 570                 575

Pro Ala Arg Val Leu Phe Lys Thr Trp Leu Glu Glu Met Tyr Lys Gln
            580                 585                 590

Val Leu Met Pro Val Val Pro Glu Ser His Arg Ala Met Tyr Ser Gln
        595                 600                 605

Thr Gly Phe Ala Thr Gln Gln Gly Pro Asn Pro Gly Ser Ile Asn Leu
    610                 615                 620

Ser Met Gly Thr Lys Val Leu Leu Arg Ala Leu Val Leu Glu Ala His
625                 630                 635                 640

Pro Asp Pro Lys Arg Val Asn Val Phe Gly Glu Arg Ser Ser Gln Glu
                645                 650                 655

Ile Met His Thr Ala Leu Gln Asn Ala Gln Ala Arg Leu Ser Gln Glu

660                 665                 670

Gln Gly Ala Gln Met Ala Arg Trp Thr Met Pro Thr Ser Val His Arg
        675                 680                 685

Phe Ser Asp Lys Asn Phe Thr Gly Thr Pro Gln Thr Met Pro Gly Asn
    690                 695                 700

Thr Phe Ala Phe Thr Gly Tyr Gln Asn Arg Gly Thr Glu Asn Asn Arg
705                 710                 715                 720

Val Val Phe Asp Ala Lys Gly Val Glu Phe Cys Asp Ala Met Pro Pro
                725                 730                 735

Gly Gln Ser Gly Phe Thr Asp Arg Asn Gly Val Arg Ser Pro His Tyr
            740                 745                 750

Glu Asp Gln Leu Lys Leu Tyr Glu Asn Phe Glu Cys Lys Thr Met Asp
        755                 760                 765

Val Thr His Ala Asp Ile Arg Arg Asn Ala Gln Ser Ser Thr Met Leu
    770                 775                 780

Leu Ile Gln Pro Gln Pro
785                 790

<210>  9

<211>  2373

<212>  DNA

<213>  Alcaligenes faecalis

<220>

<221>  CDS

<222>  (1)..(2373)

<223>  Nukleinsäure kodierend für modifizierte Pro-Penicillinamidase

<400>  9
caa gtg cag tcg gta gag gtg atg cgg gac agt tat ggc gtg ccg cac       48
Gln Val Gln Ser Val Glu Val Met Arg Asp Ser Tyr Gly Val Pro His

1                 5                   10                  15

gtc ttt gcc gac agc cac tat ggc ttg tat tac ggc tat ggt tat gcg          96
Val Phe Ala Asp Ser His Tyr Gly Leu Tyr Tyr Gly Tyr Gly Tyr Ala
                20                  25                  30

gtc gcc caa gac cgt ctg ttc cag atg gac atg gcg cgt cgc tcc ttt         144
Val Ala Gln Asp Arg Leu Phe Gln Met Asp Met Ala Arg Arg Ser Phe
            35                  40                  45

gtc ggc aca acc gcc gcc gtc tta ggc cct ggt gag caa gat gcc tac         192
Val Gly Thr Thr Ala Ala Val Leu Gly Pro Gly Glu Gln Asp Ala Tyr
        50                  55                  60

gtc aag tac gac atg cag gtg cgg cag aac ttc acc ccg gct tcc ata         240
Val Lys Tyr Asp Met Gln Val Arg Gln Asn Phe Thr Pro Ala Ser Ile
65                  70                  75                  80

cag cgg cag atc gcg gcc ttg tcc aag gat gag cgc gat att ttt cgt         288
Gln Arg Gln Ile Ala Ala Leu Ser Lys Asp Glu Arg Asp Ile Phe Arg
                85                  90                  95

ggc tat gcc gat ggc tat aac gcc tat ctg gag cag gtg cgg cgt cgc         336
Gly Tyr Ala Asp Gly Tyr Asn Ala Tyr Leu Glu Gln Val Arg Arg Arg
            100                 105                 110

cct gag ttg ctg ccc aaa gaa tat gtg gat ttt gat ttc cag ccc gag         384
Pro Glu Leu Leu Pro Lys Glu Tyr Val Asp Phe Asp Phe Gln Pro Glu
        115                 120                 125

ccg ctg acc gac ttt gat gtg gtc atg atc tgg gtg ggc tcc atg gcc         432
Pro Leu Thr Asp Phe Asp Val Val Met Ile Trp Val Gly Ser Met Ala
    130                 135                 140

aat cgc ttc tcc gac acg aat ctg gaa gtg acg gca ctg gcc atg cgt         480
Asn Arg Phe Ser Asp Thr Asn Leu Glu Val Thr Ala Leu Ala Met Arg
145                 150                 155                 160

cag tct ctg gag aaa cag cac ggc ccg gaa cga ggc cgt gcc ttg ttt         528
Gln Ser Leu Glu Lys Gln His Gly Pro Glu Arg Gly Arg Ala Leu Phe
                165                 170                 175

gat gag ctg ctg tgg atc aat gac aca aca gct ccc act acg gtt ccg         576
Asp Glu Leu Leu Trp Ile Asn Asp Thr Thr Ala Pro Thr Thr Val Pro
            180                 185                 190

gcc ccc gct gcc gag cac aag ccg cag gca caa gca ggg ggc cag gat         624
Ala Pro Ala Ala Glu His Lys Pro Gln Ala Gln Ala Gly Gly Gln Asp
        195                 200                 205

ctg gct cat gtt ggc tcg cca gta ctg gct ggc gag cta gag cgc cag         672
Leu Ala His Val Gly Ser Pro Val Leu Ala Gly Glu Leu Glu Arg Gln
    210                 215                 220

gac aag cac tgg ggc ggc cgt ggc ccg gac ttc gcg ccc aag gct agc         720
Asp Lys His Trp Gly Gly Arg Gly Pro Asp Phe Ala Pro Lys Ala Ser
225                 230                 235                 240

aac ctg tgg agc act cgc ccc gag cga gtg cag gag ggc tcg acc gta 768
Asn Leu Trp Ser Thr Arg Pro Glu Arg Val Gln Glu Gly Ser Thr Val
                245                 250                 255

ctg atc aac ggc cca cag ttt ggc tgg tac aac ccg gcc tac acc tat 816
Leu Ile Asn Gly Pro Gln Phe Gly Trp Tyr Asn Pro Ala Tyr Thr Tyr
            260                 265                 270

ggc att ggc ttg cat ggc gcc ggc ttc gat gtg gtg ggt aat acg cct 864
Gly Ile Gly Leu His Gly Ala Gly Phe Asp Val Val Gly Asn Thr Pro
        275                 280                 285

ttt gcc tat ccg atc gta ctg ttt ggc acc aat agc gag att gcc tgg 912
Phe Ala Tyr Pro Ile Val Leu Phe Gly Thr Asn Ser Glu Ile Ala Trp
    290                 295                 300

ggg gcg act gct ggc ccg caa gat gtg gtg gac ata tat cag gaa aaa 960
Gly Ala Thr Ala Gly Pro Gln Asp Val Val Asp Ile Tyr Gln Glu Lys
305                 310                 315                 320

ttg aac ccc tcg cgt gcc gat cag tac tgg ttc aac aat gcc tgg cgc 1008
Leu Asn Pro Ser Arg Ala Asp Gln Tyr Trp Phe Asn Asn Ala Trp Arg
                325                 330                 335

acg atg gag cag cgc aag gaa cgt atc cag gta cgc ggt cag gct gat 1056
Thr Met Glu Gln Arg Lys Glu Arg Ile Gln Val Arg Gly Gln Ala Asp
            340                 345                 350

cgg gaa atg acg atc tgg cgc acc gtg cac ggc cct gtg atg cag ttt 1104
Arg Glu Met Thr Ile Trp Arg Thr Val His Gly Pro Val Met Gln Phe
        355                 360                 365

gat tac gat cag ggc gcg gcg tac agc aag aaa cgc agc tgg gat ggc 1152
Asp Tyr Asp Gln Gly Ala Ala Tyr Ser Lys Lys Arg Ser Trp Asp Gly
    370                 375                 380

tat gag gtg cag tcc ttg cta gcc tgg ttg aac gtg gcc aag gcc cgc 1200
Tyr Glu Val Gln Ser Leu Leu Ala Trp Leu Asn Val Ala Lys Ala Arg
385                 390                 395                 400

aac tgg acg gag ttt ctg gat caa gcc agc aag atg gcg att tcg atc 1248
Asn Trp Thr Glu Phe Leu Asp Gln Ala Ser Lys Met Ala Ile Ser Ile
                405                 410                 415

aac tgg tac tac gcc gac aag cac ggc aat att ggt tat gtc tcg ccg 1296
Asn Trp Tyr Tyr Ala Asp Lys His Gly Asn Ile Gly Tyr Val Ser Pro
            420                 425                 430

gcc ttc ctg ccc cag cgt cct gcc gat cag gac atc cgt gtc cct gcc 1344
Ala Phe Leu Pro Gln Arg Pro Ala Asp Gln Asp Ile Arg Val Pro Ala
        435                 440                 445

aag ggg gat ggc agc atg gag tgg ctg ggc atc aag agt ttc gac gcg 1392
Lys Gly Asp Gly Ser Met Glu Trp Leu Gly Ile Lys Ser Phe Asp Ala
    450                 455                 460

att ccc aaa gcc tac aat cca ccc cag ggc tat ctg gtc aac tgg aac 1440
Ile Pro Lys Ala Tyr Asn Pro Pro Gln Gly Tyr Leu Val Asn Trp Asn

465                 470                 475                 480

aac aag cct gcg ccg gac aaa acc aat acg gat act tac tat tgg acc      1488
Asn Lys Pro Ala Pro Asp Lys Thr Asn Thr Asp Thr Tyr Tyr Trp Thr
                485                 490                 495

tat ggc gac cgc atg aat gaa ctg gtc agt cag tac cag cag aaa gac      1536
Tyr Gly Asp Arg Met Asn Glu Leu Val Ser Gln Tyr Gln Gln Lys Asp
            500                 505                 510

ctc ttc agt gtg cag gag atc tgg gag ttc aat caa aaa gcc tcc tat      1584
Leu Phe Ser Val Gln Glu Ile Trp Glu Phe Asn Gln Lys Ala Ser Tyr
        515                 520                 525

agc gat gtg aac tgg cgc tac ttc cgc cca cat ctg gaa aag ctg gcg      1632
Ser Asp Val Asn Trp Arg Tyr Phe Arg Pro His Leu Glu Lys Leu Ala
    530                 535                 540

caa cag ctg ccg gcc gac gat agc agc aag gcg gcg ctg acg atg ttg      1680
Gln Gln Leu Pro Ala Asp Asp Ser Ser Lys Ala Ala Leu Thr Met Leu
545                 550                 555                 560

ctc gcc tgg gat gga atg gaa cag gat cag gga ggg caa aat gcc gga      1728
Leu Ala Trp Asp Gly Met Glu Gln Asp Gln Gly Gly Gln Asn Ala Gly
                565                 570                 575

ccg gcg cgg gtg ctc ttc aag acc tgg ctg gaa gaa atg tac aag cag      1776
Pro Ala Arg Val Leu Phe Lys Thr Trp Leu Glu Glu Met Tyr Lys Gln
            580                 585                 590

gtc ttg atg ccg gtg gtg cct gaa tcg cat cgc gcc atg tat agc cag      1824
Val Leu Met Pro Val Val Pro Glu Ser His Arg Ala Met Tyr Ser Gln
        595                 600                 605

act ggt ttt gcc acg cag caa ggt ccc aac ccc ggt tcc atc aac ttg      1872
Thr Gly Phe Ala Thr Gln Gln Gly Pro Asn Pro Gly Ser Ile Asn Leu
    610                 615                 620

agc atg ggc acc aag gtc ttg ttg cgt gcc ttg gtg ctg gaa gcc cat      1920
Ser Met Gly Thr Lys Val Leu Leu Arg Ala Leu Val Leu Glu Ala His
625                 630                 635                 640

ccc gat ccc aag cgt gtg aat gtc ttt ggt gag cgt tcg tct cag gaa      1968
Pro Asp Pro Lys Arg Val Asn Val Phe Gly Glu Arg Ser Ser Gln Glu
                645                 650                 655

atc atg cac aca gct ttg caa aat gcg cag gcc cgc ttg agc cag gag      2016
Ile Met His Thr Ala Leu Gln Asn Ala Gln Ala Arg Leu Ser Gln Glu
            660                 665                 670

cag ggc gct cag atg gcg cgc tgg acc atg ccg acc tcc gtg cat cgt      2064
Gln Gly Ala Gln Met Ala Arg Trp Thr Met Pro Thr Ser Val His Arg
        675                 680                 685

ttc agc gac aag aac ttc acg gga acc ccg cag acg atg cct ggc aat      2112
Phe Ser Asp Lys Asn Phe Thr Gly Thr Pro Gln Thr Met Pro Gly Asn
    690                 695                 700

acc ttt gcc ttt acc ggc tat cag aat cga ggc acg gaa aat aac cgc     2160
Thr Phe Ala Phe Thr Gly Tyr Gln Asn Arg Gly Thr Glu Asn Asn Arg
705                 710                 715                 720

gtg gtg ttt gat gcc aag ggc gtg gag ttc tgc gac gcc atg ccg ccc     2208
Val Val Phe Asp Ala Lys Gly Val Glu Phe Cys Asp Ala Met Pro Pro
                725                 730                 735

ggc caa agc ggt ttc acc gac cgc aat gga gtg cgc agc ccg cat tat     2256
Gly Gln Ser Gly Phe Thr Asp Arg Asn Gly Val Arg Ser Pro His Tyr
            740                 745                 750

gag gat cag ctg aag ttg tac gag aac ttc gag tgc aag acg atg gat     2304
Glu Asp Gln Leu Lys Leu Tyr Glu Asn Phe Glu Cys Lys Thr Met Asp
        755                 760                 765

gtg acg cat gcg gac att cgt cgt aat gcg caa agc agc acg atg ctg     2352
Val Thr His Ala Asp Ile Arg Arg Asn Ala Gln Ser Ser Thr Met Leu
    770                 775                 780

ttg att cag cct cag cct taa                                         2373
Leu Ile Gln Pro Gln Pro
785                 790

<210>  10

<211>  790

<212>  PRT

<213>  Alcaligenes faecalis

<400>  10

Gln Val Gln Ser Val Glu Val Met Arg Asp Ser Tyr Gly Val Pro His
1               5                   10                  15

Val Phe Ala Asp Ser His Tyr Gly Leu Tyr Tyr Gly Tyr Gly Tyr Ala
            20                  25                  30

Val Ala Gln Asp Arg Leu Phe Gln Met Asp Met Ala Arg Arg Ser Phe
        35                  40                  45

Val Gly Thr Thr Ala Ala Val Leu Gly Pro Gly Glu Gln Asp Ala Tyr
    50                  55                  60

Val Lys Tyr Asp Met Gln Val Arg Gln Asn Phe Thr Pro Ala Ser Ile
65                  70                  75                  80

Gln Arg Gln Ile Ala Ala Leu Ser Lys Asp Glu Arg Asp Ile Phe Arg

85                  90                  95

Gly Tyr Ala Asp Gly Tyr Asn Ala Tyr Leu Glu Gln Val Arg Arg Arg
            100                 105                 110

Pro Glu Leu Leu Pro Lys Glu Tyr Val Asp Phe Asp Phe Gln Pro Glu
        115                 120                 125

Pro Leu Thr Asp Phe Asp Val Val Met Ile Trp Val Gly Ser Met Ala
    130                 135                 140

Asn Arg Phe Ser Asp Thr Asn Leu Glu Val Thr Ala Leu Ala Met Arg
145                 150                 155                 160

Gln Ser Leu Glu Lys Gln His Gly Pro Glu Arg Gly Arg Ala Leu Phe
                165                 170                 175

Asp Glu Leu Leu Trp Ile Asn Asp Thr Thr Ala Pro Thr Thr Val Pro
            180                 185                 190

Ala Pro Ala Ala Glu His Lys Pro Gln Ala Gln Ala Gly Gly Gln Asp
        195                 200                 205

Leu Ala His Val Gly Ser Pro Val Leu Ala Gly Glu Leu Glu Arg Gln
    210                 215                 220

Asp Lys His Trp Gly Gly Arg Gly Pro Asp Phe Ala Pro Lys Ala Ser
225                 230                 235                 240

Asn Leu Trp Ser Thr Arg Pro Glu Arg Val Gln Glu Gly Ser Thr Val
                245                 250                 255

Leu Ile Asn Gly Pro Gln Phe Gly Trp Tyr Asn Pro Ala Tyr Thr Tyr
            260                 265                 270

Gly Ile Gly Leu His Gly Ala Gly Phe Asp Val Val Gly Asn Thr Pro
        275                 280                 285

Phe Ala Tyr Pro Ile Val Leu Phe Gly Thr Asn Ser Glu Ile Ala Trp
    290                 295                 300

Gly Ala Thr Ala Gly Pro Gln Asp Val Val Asp Ile Tyr Gln Glu Lys
305                 310                 315                 320

Leu Asn Pro Ser Arg Ala Asp Gln Tyr Trp Phe Asn Asn Ala Trp Arg
                325                 330                 335

Thr Met Glu Gln Arg Lys Glu Arg Ile Gln Val Arg Gly Gln Ala Asp
            340                 345                 350

Arg Glu Met Thr Ile Trp Arg Thr Val His Gly Pro Val Met Gln Phe
        355                 360                 365

Asp Tyr Asp Gln Gly Ala Ala Tyr Ser Lys Lys Arg Ser Trp Asp Gly
    370                 375                 380

Tyr Glu Val Gln Ser Leu Leu Ala Trp Leu Asn Val Ala Lys Ala Arg
385                 390                 395                 400

Asn Trp Thr Glu Phe Leu Asp Gln Ala Ser Lys Met Ala Ile Ser Ile
                405                 410                 415

Asn Trp Tyr Tyr Ala Asp Lys His Gly Asn Ile Gly Tyr Val Ser Pro
            420                 425                 430

Ala Phe Leu Pro Gln Arg Pro Ala Asp Gln Asp Ile Arg Val Pro Ala
        435                 440                 445

Lys Gly Asp Gly Ser Met Glu Trp Leu Gly Ile Lys Ser Phe Asp Ala
    450                 455                 460

Ile Pro Lys Ala Tyr Asn Pro Pro Gln Gly Tyr Leu Val Asn Trp Asn
465                 470                 475                 480

Asn Lys Pro Ala Pro Asp Lys Thr Asn Thr Asp Thr Tyr Tyr Trp Thr
                485                 490                 495

Tyr Gly Asp Arg Met Asn Glu Leu Val Ser Gln Tyr Gln Gln Lys Asp
            500                 505                 510

Leu Phe Ser Val Gln Glu Ile Trp Glu Phe Asn Gln Lys Ala Ser Tyr
        515                 520                 525

Ser Asp Val Asn Trp Arg Tyr Phe Arg Pro His Leu Glu Lys Leu Ala
    530                 535                 540

Gln Gln Leu Pro Ala Asp Asp Ser Ser Lys Ala Ala Leu Thr Met Leu
545                 550                 555                 560

Leu Ala Trp Asp Gly Met Glu Gln Asp Gln Gly Gly Gln Asn Ala Gly
                565                 570                 575

Pro Ala Arg Val Leu Phe Lys Thr Trp Leu Glu Glu Met Tyr Lys Gln
            580                 585                 590

Val Leu Met Pro Val Val Pro Glu Ser His Arg Ala Met Tyr Ser Gln
        595                 600                 605

Thr Gly Phe Ala Thr Gln Gln Gly Pro Asn Pro Gly Ser Ile Asn Leu
    610                 615                 620

Ser Met Gly Thr Lys Val Leu Leu Arg Ala Leu Val Leu Glu Ala His
625                 630                 635                 640

Pro Asp Pro Lys Arg Val Asn Val Phe Gly Glu Arg Ser Ser Gln Glu
                645                 650                 655

Ile Met His Thr Ala Leu Gln Asn Ala Gln Ala Arg Leu Ser Gln Glu
            660                 665                 670

Gln Gly Ala Gln Met Ala Arg Trp Thr Met Pro Thr Ser Val His Arg
        675                 680                 685

Phe Ser Asp Lys Asn Phe Thr Gly Thr Pro Gln Thr Met Pro Gly Asn
    690                 695                 700

Thr Phe Ala Phe Thr Gly Tyr Gln Asn Arg Gly Thr Glu Asn Asn Arg
705                 710                 715                 720

Val Val Phe Asp Ala Lys Gly Val Glu Phe Cys Asp Ala Met Pro Pro
                725                 730                 735

Gly Gln Ser Gly Phe Thr Asp Arg Asn Gly Val Arg Ser Pro His Tyr
            740                 745                 750

Glu Asp Gln Leu Lys Leu Tyr Glu Asn Phe Glu Cys Lys Thr Met Asp
        755                 760                 765

Val Thr His Ala Asp Ile Arg Arg Asn Ala Gln Ser Ser Thr Met Leu
    770                 775                 780

Leu Ile Gln Pro Gln Pro
785                 790

<210>  11

<211>  111

<212>  DNA

<213>  Alcaligenes faecalis

<220>

<221>  misc_feature

<222>  (1)..(111)

<223>  Nukleinsäure kodierend für modifiziertes Linker-Peptid

<400>  11
caagcagggc cgcaggatct ggctcatgtt tcctcgccag tactggctac cgagctagag      60

cgccaggaca agcactgggg cggccgtggc ccggacttcg cgcccaaggc t              111

<210>  12

<211>  111

<212>  DNA

<213>  Alcaligenes faecalis

<220>

<221>  misc_feature

<222>  (1)..(111)

<223>  Nukleinsäure kodierend für modifiziertes Linker-Peptid

<400>  12
caagcagggg gccaggatct ggctcatgtt tcctcgccag tactggctac cgagctagag      60

cgccaggaca agcactgggg cggccgtggc ccggacttcg cgcccaaggc t              111

<210>  13

<211>  111

<212>  DNA

<213>  Alcaligenes faecalis



<220>

<221>  misc_feature

<222>  (1)..(111)

<223>  Nukleinsäure kodierend für modifiziertes Linker-Peptid



<400>  13
caagcagggg gccaggatct ggctcatgtt ggctcgccag tgctggctac cgagctagag      60

cgccaggaca agcactgggg cggccgtggc ccggacttcg cgcccaaggc t               111


<210>  14

<211>  111

<212>  DNA

<213>  Alcaligenes faecalis



<220>

<221>  misc_feature

<222>  (1)..(111)

<223>  Nukleinsäure kodierend für modifiziertes Linker-Peptid



<400>  14
caagcagggg gccaggatct ggctcatgtt ggctcgccag tactggctgg cgagctagag      60

cgccaggaca agcactgggg cggccgtggc ccggacttcg cgcccaaggc t               111


<210>  15

<211>  37

<212>  PRT

<213>  Alcaligenes faecalis



<220>

<221>  MISC_FEATURE

<222>  (1)..(37)

<223>  Wildtyp-Linker-Peptid



<400>  15

Gln Ala Gly Thr Gln Asp Leu Ala His Val Ser Ser Pro Val Leu Ala
1               5                   10                  15


Thr Glu Leu Glu Arg Gln Asp Lys His Trp Gly Gly Arg Gly Pro Asp
            20                  25                  30


Phe Ala Pro Lys Ala
        35

<210>  16

<211>  37

<212>  PRT

<213>  Alcaligenes faecalis



<220>

<221>  MISC_FEATURE

<222>  (1)..(37)

<223>  Modifiziertes Linker-Peptid



<400>  16

Gln Ala Gly Pro Gln Asp Leu Ala His Val Ser Ser Pro Val Leu Ala
1               5                   10                  15


Thr Glu Leu Glu Arg Gln Asp Lys His Trp Gly Gly Arg Gly Pro Asp
            20                  25                  30

Phe Ala Pro Lys Ala
        35

<210>  17

<211>  37

<212>  PRT

<213>  Alcaligenes faecalis

<220>

<221>  MISC_FEATURE

<222>  (1)..(37)

<223>  Modifiziertes Linker-Peptid

<400>  17

Gln Ala Gly Gly Gln Asp Leu Ala His Val Ser Ser Pro Val Leu Ala
1               5                   10                  15

Thr Glu Leu Glu Arg Gln Asp Lys His Trp Gly Gly Arg Gly Pro Asp
            20                  25                  30

Phe Ala Pro Lys Ala
        35

<210>  18

<211>  37

<212>  PRT

<213>  Alcaligenes faecalis

<220>

<221>  MISC_FEATURE

<222>  (1)..(37)

<223>  Modifiziertes Linker-Peptid

<400>  18

Gln Ala Gly Gly Gln Asp Leu Ala His Val Gly Ser Pro Val Leu Ala
1               5                   10                  15

Thr Glu Leu Glu Arg Gln Asp Lys His Trp Gly Gly Arg Gly Pro Asp
            20                  25                  30

Phe Ala Pro Lys Ala
        35

<210>  19

<211>  37

<212>  PRT

<213>  Alcaligenes faecalis

<220>

<221>  MISC_FEATURE

<222>  (1)..(37)

<223>  Modifiziertes Linker-Peptid

<400>  19

Gln Ala Gly Gly Gln Asp Leu Ala His Val Gly Ser Pro Val Leu Ala
1               5                   10                  15

Gly Glu Leu Glu Arg Gln Asp Lys His Trp Gly Gly Arg Gly Pro Asp
            20                  25                  30

Phe Ala Pro Lys Ala
        35

<210>  20

<211>  4

<212>  PRT

<213>  Alcaligenes faecalis

<220>

<221>  MISC_FEATURE

<222>  (1)..(4)

<223>  Fragment eines modifizierten Linker-Peptids

<400>  20

Gln Ala Gly Pro
1

<210>  21

<211>  11

<212>  PRT

<213>  Alcaligenes faecalis

<220>

<221>  MISC_FEATURE

<222>  (1)..(11)

<223>  Fragment eines modifizierten Linker-Peptids

<400>  21

Gln Ala Gly Pro Gln Asp Leu Ala His Val Ser
1               5                   10

<210>  22

<211>  17

<212>  PRT

<213>  Alcaligenes faecalis

<220>

<221>  MISC_FEATURE

<222>  (1)..(17)

<223>  Fragment eines modifizierten Linker-Peptids

<400>  22

Gln Ala Gly Pro Gln Asp Leu Ala His Val Ser Ser Pro Val Leu Ala
1               5                   10                  15

Thr

<210>  23

<211>  20

<212>  PRT

<213>  Alcaligenes faecalis

<220>

<221>  MISC_FEATURE

<222>  (1)..(20)

<223>  Fragment eines modifizierten Linker-Peptids

<400>  23

Gln Ala Gly Pro Gln Asp Leu Ala His Val Ser Ser Pro Val Leu Ala
1               5                   10                  15

Thr Glu Leu Glu
            20

<210>  24

<211>  29

<212>  PRT

<213>  Alcaligenes faecalis

<220>

<221>  MISC_FEATURE

<222>  (1)..(29)

<223>  Fragment eines modifizierten Linker-Peptids

<400>  24

Gln Ala Gly Pro Gln Asp Leu Ala His Val Ser Ser Pro Val Leu Ala
1               5                   10                  15

Thr Glu Leu Glu Arg Gln Asp Lys His Trp Gly Gly Arg
            20                  25

<210>  25

<211>  4

<212>  PRT

<213>  Alcaligenes faecalis

<220>

<221>  MISC_FEATURE

<222>  (1)..(4)

<223>  Fragment eines modifizierten Linker-Peptids

<400>  25

Gln Ala Gly Gly
1

<210>  26

<211>  11

<212>  PRT

<213>  Alcaligenes faecalis

<220>

<221>  MISC_FEATURE

<222>  (1)..(11)

<223>  Fragment eines modifizierten Linker-Peptids

<400>  26

Gln Ala Gly Gly Gln Asp Leu Ala His Val Ser
1               5                   10

<210>  27

<211>  17

<212>  PRT

<213>  Alcaligenes faecalis

<220>

<221>  MISC_FEATURE

<222>  (1)..(17)

<223>  Fragment eines modifizierten Linker-Peptids

<400>  27

Gln Ala Gly Gly Gln Asp Leu Ala His Val Ser Ser Pro Val Leu Ala
1               5                   10                  15

Thr

<210>  28

<211>  20

<212>  PRT

<213>  Alcaligenes faecalis

<220>

<221>  MISC_FEATURE

<222>  (1)..(20)

<223>  Fragment eines modifizierten Linker-Peptids

<400>  28

Gln Ala Gly Gly Gln Asp Leu Ala His Val Ser Ser Pro Val Leu Ala
1               5                   10                  15

Thr Glu Leu Glu
            20

<210>  29

<211>  29

<212>  PRT

<213>  Alcaligenes faecalis

<220>

<221>  MISC_FEATURE

<222>  (1)..(29)

<223>  Fragment eines modifizierten Linker-Peptids

<400>  29

Gln Ala Gly Gly Gln Asp Leu Ala His Val Ser Ser Pro Val Leu Ala
1               5                   10                  15

Thr Glu Leu Glu Arg Gln Asp Lys His Trp Gly Gly Arg
            20                  25

<210>  30

<211>  11

<212>  PRT

<213>  Alcaligenes faecalis

<220>

<221>  MISC_FEATURE

<222>  (1)..(11)

<223>  Fragment eines modifizierten Linker-Peptids

<400>  30

Gln Ala Gly Gly Gln Asp Leu Ala His Val Gly
1               5                   10

<210>  31

<211>  17

<212>  PRT

<213>  Alcaligenes faecalis

<220>

<221>  MISC_FEATURE

<222>  (1)..(17)

<223>  Fragment eines modifizierten Linker-Peptids

<400>  31

Gln Ala Gly Gly Gln Asp Leu Ala His Val Gly Ser Pro Val Leu Ala
1               5                   10                  15

Thr

<210>  32

<211>  20

<212>  PRT

<213>  Alcaligenes faecalis

<220>

<221>  MISC_FEATURE

<222>  (1)..(20)

<223>  Fragment eines modifizierten Linker-Peptids

<400>  32

Gln Ala Gly Gly Gln Asp Leu Ala His Val Gly Ser Pro Val Leu Ala
1               5                   10                  15

Thr Glu Leu Glu
            20

<210>  33

<211>  29

<212>  PRT

<213>  Alcaligenes faecalis

<220>

<221>  MISC_FEATURE

<222>  (1)..(29)

<223>  Fragment eines modifizierten Linker-Peptids

<400>  33

Gln Ala Gly Gly Gln Asp Leu Ala His Val Gly Ser Pro Val Leu Ala
1               5                   10                  15

Thr Glu Leu Glu Arg Gln Asp Lys His Trp Gly Gly Arg
            20                  25

<210>  34

<211>  17

<212>  PRT

<213>  Alcaligenes faecalis

<220>

<221>  MISC_FEATURE

<222>  (1)..(17)

<223>  Fragment eines modifizierten Linker-Peptids

<400>  34

Gln Ala Gly Gly Gln Asp Leu Ala His Val Gly Ser Pro Val Leu Ala
1               5                   10                  15

Gly

<210>  35

<211>  20

<212>  PRT

<213>  Alcaligenes faecalis

<220>

<221>  MISC_FEATURE

<222>  (1)..(20)

<223>  Fragment eines modifizierten Linker-Peptids

<400>  35

Gln Ala Gly Gly Gln Asp Leu Ala His Val Gly Ser Pro Val Leu Ala
1               5                   10                  15

Gly Glu Leu Glu
            20

<210>  36

<211>  29

<212>  PRT

<213>  Alcaligenes faecalis

<220>

<221>  MISC_FEATURE

<222>  (1)..(29)

<223>  Fragment eines modifizierten Linker-Peptids

<400>  36

Gln Ala Gly Gly Gln Asp Leu Ala His Val Gly Ser Pro Val Leu Ala
1               5                   10                  15

Gly Glu Leu Glu Arg Gln Asp Lys His Trp Gly Gly Arg
            20                  25

<210>  37

<211>  4

<212>  PRT

<213>  Alcaligenes faecalis

<220>

<221>  MISC_FEATURE

<222>  (1)..(4)

<223>  Wildtyp-Linkerfragment

<400>  37

Gln Ala Gly Thr
1

<210>  38

<211>  11

<212>  PRT

<213>  Alcaligenes faecalis

<220>

<221>  MISC_FEATURE

<222>  (1)..(11)

<223>  Wildtyp-Linkerfragment

<400>  38

Gln Ala Gly Thr Gln Asp Leu Ala His Val Ser
1               5                   10

<210>  39

<211>  17

<212>  PRT

<213>  Alcaligenes faecalis

<220>

<221>  MISC_FEATURE

<222>  (1)..(17)

<223>  Wildtyp-Linkerfragment

<400>  39

Gln Ala Gly Thr Gln Asp Leu Ala His Val Ser Ser Pro Val Leu Ala
1               5                   10                  15

Thr

<210>  40

<211>  20

<212>  PRT

<213>  Alcaligenes faecalis

<220>

<221>  MISC_FEATURE

<222>  (1)..(20)

<223>  Wildtyp-Linkerfragment

<400>  40

Gln Ala Gly Thr Gln Asp Leu Ala His Val Ser Ser Pro Val Leu Ala
1               5                   10                  15

Thr Glu Leu Glu
            20

<210>  41

<211>  29

<212>  PRT

<213>  Alcaligenes faecalis

<220>

<221>  MISC_FEATURE

<222>  (1)..(29)

<223>  Wildtyp-Linkerfragment

<400>  41

Gln Ala Gly Thr Gln Asp Leu Ala His Val Ser Ser Pro Val Leu Ala
1               5                   10                  15

Thr Glu Leu Glu Arg Gln Asp Lys His Trp Gly Gly Arg
            20                  25